(19) Europäisches Patentamt
European Patent Office
Office européen des brevets

(11) Publication number: **0 010 297**
**B1**

(12) **EUROPEAN PATENT SPECIFICATION**

(45) Date of publication of patent specification: **13.04.83**

(51) Int. Cl.³: C 07 H 17/08, A 61 K 31/70

(21) Application number: **79104014.0**

(22) Date of filing: **18.10.79**

# (54) Derivatives of polyene macrolide antibiotics containing an amino sugar moiety, process for the preparation thereof, and pharmaceutical compositions containing them.

(30) Priority: **23.10.78 US 953824**

(43) Date of publication of application: **30.04.80 Bulletin 80/9**

(45) Publication of the grant of the patent: **13.04.83 Bulletin 83/15**

(84) Designated Contracting States: **AT BE CH DE FR GB IT NL SE**

(56) References cited:
**US - A - 3 244 590**
**US - A - 4 041 232**

(73) Proprietor: **SCHERING CORPORATION**
**2000 Galloping Hill Road**
**Kenilworth, New Jersey 07033 (US)**

(72) Inventor: **Wright, John Jessen**
**4 Todd Terrace**
**Cedar Grove New Jersey 07009 (US)**

(74) Representative: **Antony, Fritz, Dr. et al,**
**SCHERICO LTD. P.O. Box 601 Winkelriedstrasse 35**
**CH-6002 Lucerne (CH)**

**EP 0 010 297 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Derivatives of polyene macrolide antibiotics containing an amino sugar moiety, process for the preparation thereof, and pharmaceutical compositions containing them

This invention relates to derivatives of polyene macrolide antibiotics containing an amino sugar moiety, process for the preparation thereof, and pharmaceutical compositions containing them. It has been established that compounds of this invention show antifungal activity and are useful in the treatment of fungal infections.

Polyene macrolide antibiotics containing an amino sugar moiety are known in the art. In general they are useful in the treatment of mycosis, benign prostatic hypertrophy and elevated blood cholesterol. Among such known polyene macrolides are amphotericin B, candicidin and nystatin which are used as anti-fungal agents. The most commonly known and used agent is amphotericin B in the form of its sodium desoxycholate complex, which is sold under the registered trademark FUNGIZONE. Lack of water solubility, poor stability, and toxic properties have hindered other polyene macrolides achieving an important place in therapy.

Attempts have been made to improve the usefulness of amphotericin B. Straight chain alkyl esters of amphotericin B and a number of other polyene antibiotic have been reported. The reports indicate that these esters and their salts generally have reduced toxicity compared with the parent polyene antibiotics, but at the same time they also have reduced activity. N-Acyl derivatives of these compounds have also been prepared and, again, although they have been found to be less toxic than their parent polyene antibiotics, they have also been found to be less potent. N-Glycosyl derivatives of certain polyene antibiotics have also been reported and these appear to be equipotent or more potent than their parents and about as toxic. According the prior art it was not possible so far, to produce a less toxic but potent systemic anti-fungal agent.

The novel compounds of this invention are the polyene macrolide antibiotic derivatives of the general formula

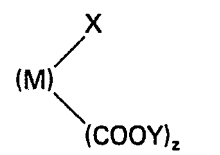 (I)

wherein M is a polyene macrolide antibiotic moiety containing an amino sugar moiety, the amino group of which is substituted by X, wherein X is

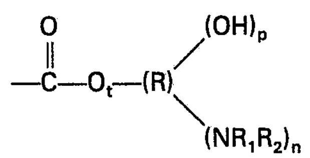 (II),

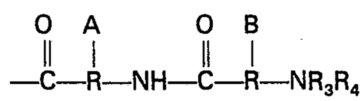 (III),

or

$-R_5$,

wherein A and B independently of each other are hydrogen or $NR_3R_4$ or one of A and B is hydrogen and the other is

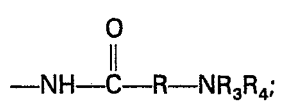

R is a saturated hydrocarbon group having 1 to 10 carbon atoms; $R_1$ and $R_2$ independently of each other are hydrogen or a straight chain alkyl group having 1 to 3 carbon atoms or one of $R_1$ and $R_2$ is hydrogen and the other a straight chain alkyl group having 1 to 3 carbon atoms substituted by an $NH_2$ group; $R_3$ and $R_4$ independently of each other are hydrogen or a straight chain alkyl group containing 1 to 3 carbon atoms; $R_5$ is histidyl, tyrosyl, tryptophyl, arginyl, phenylalanyl or prolyl or an

$(\alpha)-N-[(NH_2)_m-(R)-CO]$-derivative

of histidyl, tyrosyl, tryptophyl, phenylalanyl or prolyl; m is 1 or 2; n is 1 to 3, t is zero or 1, and when t is zero p is zero to 4 and when t is 1 p is zero; with the proviso that not more than one hetero atom is

attached to the same carbon atom in any one of R, $R_1$ and $R_2$ and that in those compounds containing more than one R-group, these R-groups may be of the same or different chain length;

z is zero or 1, and

Y is hydrogen, an alkyl group containing 1 to 10 carbon atoms, allyl, propargyl, benzyl or phenylpropargyl; and the pharmaceutically acceptable acid addition salts of all the foregoing compounds. Preferably these derivatives are derived from a polyene macrolide antibiotic of the general formula

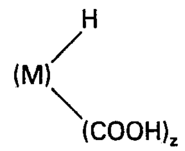 (IV),

wherein z is zero or 1, which is amphotericin B, ascocin, aureofacin, aureofungin A, aureofungin B, candicidin, candidin, eurotin A, flavomycin, fulvomicin A, fulvomicin B, fulvomicin C, fungimycin, hamycin A, lucensomycin, levorin, mycoheptin, nystatin A, partricin, pimaricin, polyfungin, rimocidin, trichomycin A, vacidin A, DJ-400 $B_1$, DJ-400 $B_2$, 67-121A, 67-121B, 67-121C, PA 150, PA616, or 2814 H, of which pimaricin and 67-121C and especially amphotericin B are most preferred. The amino sugar moiety generally is mycosamine or perosamine.

The listed macrolide antibiotics are known in the art. For example aureofungin A and B, fulvomycin A, B and C, polyfungin B, vacidin A, DJ-400$B_1$ and $B_2$, 67-121A, B and C, PA 150, PA 616, 2814H are disclosed in a review article by Doctor Allan Mallams in the Kirk-Othmar Encyclopedia of Technology, Vol. 3, 3rd edition, pages 21—47.

Some of the polyene macrolide antibiotics contain aromatic moieties on the macrolide ring and these aromatic rings may also contain an amine group; however, under the reaction conditions of this invention, these amine groups are not involved.

As stated before R is a saturated hydrocarbon having 1 to 10 carbon atoms, said hydrocarbon may be a straight or branched chain alkylene group, such as for example methylene, ethylene, *n*-propylene, isopropylene, *n*-butylene, isobutylene, *tert.*-butylene, *β*-methylpropylene, pentylene, hexylene, heptylene, octylene, nonylene, decylene and the like.

Preferably R is a straight chain alkylene group especially a group containing 1 to 5 carbon atoms.

Those derivatives are preferred wherein X is

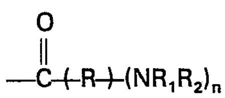 (V),

wherein n is 1 or preferably 2 and R, $R_1$ and $R_2$ are as defined above.

Preferred are those derivatives wherein $R_1$, $R_2$, $R_3$ and $R_4$ are hydrogen, and, provided that the compound contains two $NH_2$ groups, one of the amino groups is terminal.

As is apparent from the above, substituent X (formula I) may consist of one, two or three aminoacyl groups, preferably allanyl, glycyl, histidyl, tryptophyl, isoleucyl, leucyl, lysyl, norvalyl, ornithyl, phenylalanyl, prolyl, seryl, tyrosyl or valyl. X may contain identical or different amino acyl groups, so that X is glycyl-glycyl, glycyl-lysyl, leucyl-leucyl, alanyl-lysyl, histidyl-lysyl, and the like.

Preferably X is lysyl or ornithyl.

In those instances where the group X has one or more chiral centers, the diastereomeric derivatives of formula I may be used separately or in admixture. For example, the nature of the synthesis may result in formation of a N-[D]-lysyl, a N-[L]-lysyl, or a N-[D,L]-lysyl amphotericin B, the latter being an equal mixture of the first two.

Derivatives of polyene macrolide antibiotics containing a carboxylic acid function (i.e. compounds of formula I wherein z is 1) are preferred in the form of their esters. Derivatives are preferred wherein Y is an alkyl group containing 1 to 4 carbon atoms, especially those wherein Y is methyl.

The pharmaceutically acceptable acid addition salts of this invention can be derived from inorganic acids, such as for example hydrochloric, sulfuric, and phosphoric acid, or from organic acids such as for example formic, maleic and tartaric acid. The physical embodiments of the salts are characterized by being colorless to yellow/orange solids (dependent on the degree of unsaturation of the polyene) which are soluble in water, sparingly soluble in most polar organic solvents and insoluble in most non-polar organic solvents.

Of the compounds of this invention the following are of special interest:

N-D-lysyl amphotericin B methyl ester, N-D-ornithyl amphotericin B methyl ester, N-D-lysyl pimaricin methyl ester, N-glycyl 67-121C, N-glycyl amphotericin B methyl ester, N-D-2,4-diaminobutyryl amphotericin B methyl ester, N-*β*-alanyl amphotericin B methyl ester, N-(aminoethylglycyl) amphotericin B, N-(aminoethylglycol) amphotericin B methyl ester, N-D-lysyl amphotericin B, N-L-lysyl amphotericin B, N-L-lysyl amphotericin B methyl ester, N-L-ornithyl amphotericin B methyl ester, N-D-ornithyl amphotericin B propargyl ester, N-(2-aminoethoxycarbonyl) amphotericin B methyl ester, N-L-histidyl amphotericin B methyl ester, N-D-lysyl amphotericin B propargyl ester, N-histidyl-lysyl amphotericin B methyl ester, N-D-ornithyl-amphotericin B, N-L-ornithyl amphotericin B, and the pharmaceutically acceptable acid addition salts, especially hydrochloride salts, thereof.

The compounds of this invention are useful in the treatment of mycosis, benign prostatic hypertrophy and elevated blood cholesterol, as the known polyene macrolide antibiotics. The compounds of this invention are especially useful as anti-fungal agents. Surprisingly, in tests compounds of this invention have shown to be less toxic and more active than the respective known polyene macrolide antibiotics.

The compounds of this invention thus can be used to effectively combat fungus infections of the following species: Histoplasma capsulatum, Coccidiodes immitis, Candida spp., Blastomyces dermatitidis, Rhodotorula, Cryptococcus neoformans, Sporotrichum schenckii, Mucor nucedo, Aspergillus fumigatus, Trichophyton spp., Microsporum spp. and Epidermophyton. Additionally, the compounds have been found to be active fungicidally rather than fungistatically.

The properties of the compounds of this invention can be illustrated by the following test results:

TABLE 1
In vitro activity

| Organisms | Average MIC's (mcg/ml, 48 hrs.) S.D.B. pH 5.8 | | | |
|---|---|---|---|---|
| Compound: | A | B | C | D |
| 64 strains *Candida albicans* | 0.02 | 0.03 | 0.04 | 0.08 |
| 13 strains non-albicans yeasts | 0.02 | 0.02 | 0.05 | 0.10 |
| 2 filamentous fungi | 0.04 | 0.10 | 0.11 | 0.23 |

TABLE 2
Effective dose (ED, mg/kg) needed to Cure[a] Mice infected with various strains of Candida Albicans and $LD_{50}$-I.V. Mice (mg/kg)

| Compound | ED [mg/kg] C.albicans Strains | | | | $LD_{50}$ [mg/kg] |
|---|---|---|---|---|---|
| | Wisconsin | C-17 | C-60 | Sparks | |
| D | 0.7 | 3 | 1 | 1.2 | 4 |
| C | ≥20 | | | | 100 |
| A | 0.7 | 3 | 1.5 | 2.6 | 35—40 |
| B | 0.7 | 3 | 1.5 | 2.6 | 35—40 |

[a]Reduce counts to below $10^5$/mouse kidneys.

*Compound A:* N-D-lysyl amphotericin B methyl ester dihydrochloride.
*Compound B:* N-D-ornithyl amphotericin B methyl ester dihydrochloride.
*Compound C:* amphotericin B methyl ester.
*Compound D:* fungizone® (=amphotericin B as the sodium desoxycholate complex).

In general, the compounds of this invention and their pharmaceutically acceptable acid addition salts may be administered orally, e.g. compounded in the form of tablets, capsules, elixirs or the like, or they may be applied topically, e.g. in the form of ointments, lotions, creams or gels. Preferantially, they may be administered parenterally via intramuscular, intravenous, subcutaneous, intralesional and intrasternal injection. The injectable solution will usually be administered at from about 1 mg to 5 mgs of compound per kilogram of body weight per day. Pharmaceutical carriers useful in the preparation of the foregoing formulations will include, for example, such substances as water, oils, fats, waxes, polyesters, alcohols, polyols and the like.

The present invention includes within its scope the method for treating fungal infections which comprises administering to a mammal infected with a susceptible fungus a non-toxic fungicidally effective amount of a compound of this invention. Also included within the inventive concept are pharmaceutical compositions comprising a non-toxic, fungicidally effective amount of a compound of this invention together with a non-toxic, pharmaceutically acceptable carrier.

The compounds of this invention can be prepared according to known methods, especially according to methods known from peptide synthesis. In these methods the polyene macrolide

antibiotics containing an aminosugar moiety are used as starting material, especially amphotericin B, ascocin, aureofacin, aureofungin A, aureofungin B, candicidin, candidin, eurotin A, flavomycin, fulvomicin A, fulvomicin B, fulvomicin C, fungimycin, hamycin A, leucensomycin, levorin, mycoheptin, nystatin A, partricin, pimaricin, polyfungin, rimocidin, trichomycin A, vacidin A, DJ-400 $B_1$, DJ-400 $B_2$, 67-121A, 67-121B, 67-121C, PA 150, PA 616 and 2814H.

The polyene macrolides are highly insoluble compounds so that when they are reacted with other compounds, various polar organic solvents must be used as, for example, dimethylformamide, dimethylsulfoxide, and hexamethylphosphorictriamide. In reactions wherein acylation of the polyene's hydroxyl group(s) has to be prevented a small amount of methanol to any of the polar solvents is usually added.

A. For the preparation of derivatives of polyene macrolide antibiotics of the general formula

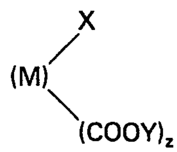 (I),

wherein M is a polyene macrolide antibiotic moiety containing an amino sugar moiety, the amino group of which is substituted by X, wherein X is

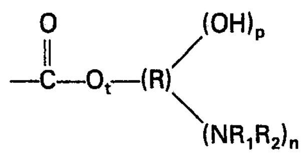 (II),

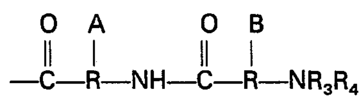 (III),

or

$—R_5$,

wherein A and B independently of each other are hydrogen or $NR_3R_4$ or one of A and B is hydrogen and the other is

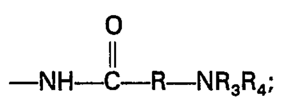

R is a saturated hydrocarbon group having 1 to 10 carbon atoms; $R_1$ and $R_2$ independently of each other are hydrogen or a straight chain alkyl group having 1 to 3 carbon atoms or one of $R_1$ and $R_2$ is hydrogen and the other a straight chain alkyl group having 1 to 3 carbon atoms substituted by an $NH_2$ group; $R_3$ and $R_4$ independently of each other are hydrogen or a straight chain alkyl group containing 1 to 3 carbon atoms; $R_5$ is histidyl, tyrosyl, tryptophyl, arginyl, phenylalanyl or prolyl or an

$(\alpha)—N—[(NH_2)_m—(R)—CO]$-derivative

of histidyl, tyrosyl, tryptophyl, phenylalanyl or prolyl; m is 1 or 2; n is 1 to 3, t is zero or 1, and when t is zero p is zero to 4 and when t is 1 p is zero; with the proviso that not more than one hetero atom is attached to the same carbon atom in any one of R, $R_1$ and $R_2$ and that in those compounds containing more than one R-group, these R-groups may be of the same or different chain length;

z is zero or 1, and

Y is hydrogen, an alkyl group containing 1 to 10 carbon atoms, allyl, propargyl, benzyl or phenylpropargyl, with the proviso that, when X is hydrogen, z must be 1 and Y allyl, propargyl, benzyl or phenylpropargyl; a polyene macrolide antibiotic of the general formula

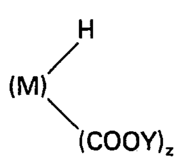 (VI),

wherein M, Y and z are as defined above, is reacted with a reactive derivative of a compound of the general formula HOX′(VII), wherein X′ is defined as group X above with the exception that the amino groups contained therein are protected. This reaction is followed by the removal of the protecting group(s) and, if desired, esterification of compounds containing a carboxyl group. For the preparation of compounds containing an esterified carboxyl group it is preferred, however, to carry out the said esterification before removing the protecting group(s).

The process may be outlined by the following reaction scheme:

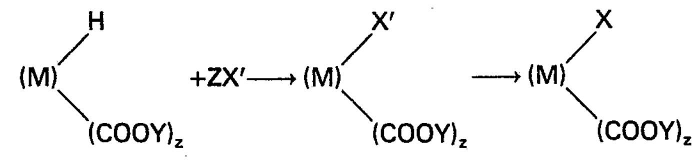

(VI) (VIII) (IX) (I)

In the formulae of this reaction scheme M, Y, z and X′ are as defined above and Z is an eliminable group, such as for example N-hydroxysuccinimidyl, N-hydroxyphthalimidyl or an acyl imidazolyl. Preferably Z is N-hydroxysuccinimidyl.

The amino groups contained in compound ZX′ are protected by easily removable groups, such as for example trifluoroacetyl, 2,2,2-trichloroethoxycarbonyl and fluorenemethoxycarbonyl, the latter group being preferred.

The reaction of the compounds of formula VI and VIII is carried out in a polar organic solvent such as dimethylformamide, dimethylsulfoxide, hexamethylphosphoric triamide or mixtures of these solvents. In reactions wherein acylation of possible hydroxyl group(s) of the polyene antibiotic has to be prevented a small amount of methanol can be added. Several other means can be used to support this reaction, such as performing the reaction under nitrogen and/or addition of triethylamine.

The compound ZX′ can be prepared by known methods. For the preparation of compounds of formula I wherein X is the group

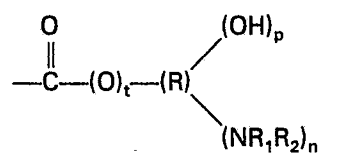

(II)

wherein t is one, p is zero and R, $R_1$, $R_2$ and n are as defined above, ZX′ (VIII) can be prepared by reacting an appropriate N-protected aminoalkanol with phosgene and then with preferably N-hydroxysuccinimide. For the preparation of all other compounds of formula I ZX′ can be prepared by reacting the appropriate N-protected amino acid with preferably N-hydroxysuccinimide and dicyclohexylcarbodiimide.

The reaction conditions under which the protecting groups of the amino groups are removed in this process are known in the art and depend on the kind of protecting group.

Compounds of formula I and IX containing a free carboxy group, (wherein z is one and Y is hydrogen) can be esterified by reaction with a reactive derivative of the appropriate hydrocarbon. The compounds of formula I, having a free carboxyl group and at least one free amino group, can be esterified for example by reaction with the appropriate diazo derivative of the hydrocarbon such as diazomethane, diazoethane, diazopropane or diazobutane (e.g. as described in U.S. Patent No. 3.936.526 and 4.038.382). The compounds of formula Ix, which are compounds containing one or more protected amino groups can be reacted with alkylating agents such as for example methyl iodide, methyltosylate, ethyliodide, or with allyliodide, propargyl bromide methyl sulfate and phenylpropargyl bromide. In this alkylation reaction it is preferred to use compounds of formula IX wherein the amino group(s) is(are) protected by fluoroenemethyloxycarbonyl.

B. For the preparation of a derivative of a polyene macrolide antibiotic of the general formula

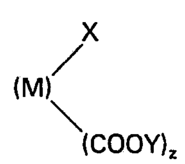

(I)

wherein M is a polyene macrolide antibiotic moiety containing an amino sugar moiety, the amino group of which is substituted by X, wherein X is

$$—CO—CH_2—NHR_1 \qquad (X),$$

wherein $R_1$ is hydrogen, or a straight chain alkyl group having 1 to 3 carbon atoms which may be substituted by an $NH_2$ group; z is zero or 1, and Y is hydrogen, an alkyl group containing 1 to 10 carbon atoms, allyl, propargyl, benzyl or phenylpropargyl, a N-iodoacetyl derivative of the polyene macrolide antibiotic is reacted with an amine of the general formula $H_2NR_1$ (XI), wherein $R_1$ is as defined above and, if desired, esterified, provided the macrolide contains a carboxy group. The reaction is carried out in a polar solvent such as for example dimethylformamide, dimethylsulfoxide and hexamethylphosphorictriamide. Methanol may be added to the solvent. By this process derivatives such as for example the N-glycyl, N-methylglycyl and N-aminoethylglycyl derivatives of the polyene macrolide antibiotics can be prepared. The so obtained compounds can be esterified as described in process A.

The N-iodoacetyl derivatives of the polyene macrolide antibiotics can be prepared by reacting the relevant macrolide with iodoacetic anhydride in a polar organic solvent.

The so obtained products may be subjected to an after treatment such as transformation into the pharmaceutically acceptable acid addition salt. The salts are made according to known procedures such as by neutralizing the free base with the appropriate acid, usually to about pH 5. The acid addition salts may be mono- or di- in nature depending on the number of free amine groups in the compound, the amount of acid used and whether or not the carboxy group possibly contained in the base is esterified.

The above processes are exemplified by the following Examples.

Example 1

N-(2-Aminoethoxycarbonyl)Amphotericin B

A. N-Trifluoroacetyl-ethanolamine

Ethanolamine (12 g) is dissolved in methanol (50 ml) and *S*-ethylthiotrifluoroacetate (35 g) is added dropwise. The reaction is permitted to stand overnight irrigated with a stream of argon to remove ethanethiol. The excess solvent is removed *in vacuo* to leave the title product.

B. 2-N-Trifluoroacetylaminoethoxycarbonyloxy succinimide

N-Trifluoroacetyl-ethanolamine (7.8 g) in dichloromethane (100 ml) is treated with phosgene (15 g) in dichloromethane (120 ml). Triethylamine (6 ml) is added dropwise with stirring.

After four hours at room temperature excess phosgene is removed by the rapid passage of nitrogen and the solution is filtered and the solvent removed *in vacuo*. The residue is dissolved in dichloromethane (100 ml) and *N*-hydroxysuccinimide (5.8 g) and pyridine (12 g) are added. After 18 hours the solvent is removed under vacuum to provide the named compound as a residue which is used without further purification.

C. N-(2-N'-Trifluoroacetylaminoethoxycarbonyl) amphotericin B

Amphotericin B (2 g) in 27.5 ml of a 9:1 dimethylsulfoxide: methanol mixture is treated with 2-N-trifluoroacetylaminoethoxycarbonyloxysuccinimide (3.9 g) dissolved in a minimum amount of tetrahydrofuran, added in portions over 7.5 hours. Triethylamine (327 mg) is added and the mixture is stirred for 2.5 hours. The mixture is then diluted with 25 ml of methanol and added dropwise to 600 ml of ether. Filtration of the yellow precipitate, washing with ether and drying gives the named compound: $\nu$ max (KBr) 1715 $cm^{-1}$; $\lambda$ max. (20% aqueous tetrahydrofuran), 348 nm ($E^1_{1\%}$=263), 367 nm ($E^1_{1\%}$=563), 386 nm ($E^1_{1\%}$=946), 410 nm ($E^1_{1\%}$=1056).

D. N-(2-Aminoethoxycarbonyl) amphotericin B

N-(2-N'-trifluoroacetylaminoethoxycarbonyl)amphotericin B (1.985 g) in 60 ml of a 1:1 dimethylformamide:concentrated ammonium hydroxide mixture is stirred 9.5 hours with monitoring by thin layer chromatography (2:2:1 chloroform:methanol:water, lower phase). Dilution with 60 ml methanol and dropwise addition to excess ether yields a yellow precipitate which is filtered, washed with ether and dried. The precipitate is dissolved in 10 ml dimethylsulfoxide and chromatographed on 250 g $SiO_2$ eluted with the lower phase of a 2:2:1 chloroform:methanol:water mixture, to obtain the title compound: $[\alpha]^{26}_D$+213.6° (DMSO); $\lambda$ max (20% aqueous tetrahydrofuran) 348 nm ($E^1_{1\%}$=310), 367 nm ($E^1_{1\%}$=680), 386 nm ($E^1_{1\%}$=1140), 410 nm ($E^1_{1\%}$=1270).

Example 2

N-Glycyl amphotericin B methyl ester hydrochloride

A. N-Trifluoroacetylglycyloxy succinimide

*N*-Trifluoroacetylglycine (684 mg) and N-hydroxysuccinimide (480 mg) in 5 ml of dry $CH_2Cl_2$ at 50°C are treated with a solution of 915 mg dicyclohexylcarbodiimide (4.43 mmol) in 5 ml of $CH_2Cl_2$. The mixture is stirred at room temperature overnight, is then filtered and evaporated to produce the named compound which can be used in subsequent reactions without further purification.

B. N-(N'-Trifluoroacetylglycyl) amphotericin B

Amphotericin B (500 mg) in 5.5 ml 9:1 dimethylsulfoxide:methanol with 246 mg of the product of step A is stirred at 25°C for three hours. The mixture is diluted with 5 ml methanol and added dropwise to 100 ml ether. The yellow precipitate is filtered, washed with ether and dried to give the named compound; $\nu$ max (KBr) 1710, 1680 $cm^{-1}$.

C. N-Glycyl amphotericin B

N-(N'-trifluoroacetylglycyl)amphotericin B (372 mg) in 13 ml 40% concentrated solution of ammonium hydroxide in dimethylformamide is stirred at room temperature with careful monitoring by thin layer chromatography ($SiO_2$ eluted with the lower phase of a 2:2:1 chloroform:methanol:water mixture). After 5 hours the mixture is reduced to half volume *in vacuo*, diluted with an equal volume of methanol and added dropwise to excess ether. The resulting yellow precipitate is filtered, triturated twice with ethanol and once with ether and dried to produce the named compound.

D. N-Glycyl amphotericin B methyl ester

A solution of N-glycyl amphotericin B (75 mg) in 2 ml dimethylsulfoxide is treated with ethereal diazomethane. After evolution of nitrogen has ceased, the reaction is judged complete on the basis of thin layer chromatography ($SiO_2$ eluted with the lower phase of either a 2:2:1 chloroform:methanol:water or 1:1:1 chloroform:methanol:concentrated ammonium hydroxide mixture). The mixture is diluted with 2 ml methanol and added dropwise to excess ether. The resulting yellow precipitate is filtered, washed with ether and dried to produce the named compound: $\nu$ max (KBr): 1710, 1660 $cm^{-1}$.

E. N-Glycyl amphotericin B methyl ester hydrochloride

To N-glycyl amphotericin B methyl ester dissolved in aqueous tetrahydrofuran (1:1) is added 0.1 N hydrochloric acid until a pH of 5 is reached. The solution is filtered through a 0.45 $\mu$ micropore filter. The filtrate is lyophilized to yield the title compound: $[\alpha]_D^{26}$–580° ($H_2O$); $\lambda$ max (20% aqueous tetrahydrofuran (367 nm ($E_{1\%}^1$=800), 387 nm ($E_{1\%}^1$=1280), 411 nm ($E_{1\%}^1$=1430).

Example 3

N-D,L-Lysyl amphotericin B methyl ester

A. N-(N',N''-Bis-trifluoroacetyl-L-lysyl)amphotericin B

N,N'-bis-trifluoroacetyl-L-lysine is treated with N-hydroxysuccinimide according to Example 2A to obtain N,N'-bis-trifluoroacetyl-L-lysyloxy succinimide; $[\alpha]_D^{26}$–31.4° (EtOH); $\nu$ max (KBr) 1810, 1790, 1740, 1700 $cm^{-1}$. This product (473 mg) is added under nitrogen to amphotericin B (500 mg) in 5 ml of a 9:1 dimethylsulfoxide:methanol mixture. After 3.5 hours, 6 ml of methanol is added and the mixture is added dropwise to excess ether. A fine, yellow precipitate is allowed to settle with cooling overnight. Filtration, washing with ether and drying gives the named compound: $[\alpha]_D^{26}$+263.2 (dimethylsulfoxide); $\nu$ max (KBr) 1710 $cm^{-1}$.

B. N-D,L-Lysyl amphotericin B

N-(N',N''-bis-trifluoroacetyl-L-lysyl)amphotericin B (418 mg) in 30 ml of a 1:1 concentrated ammonium hydroxide:methanol mixture is stirred with careful monitoring by thin layer chromatography, ($SiO_2$ eluted with the lower phase of a 2:2:1 chloroform:methanol:water mixture). After 11.5 hours the mixture is added dropwise to excess ether. The yellow precipitate is filtered and washed successively with ethanol and ether, providing the named compound: $[\alpha]_D^{26}$+293.4° (DMSO); $\nu$ max (KBr) 3400, 1710—1630 $cm^{-1}$; $\lambda$ max (20% aqueous tetrahydrofuran), 348 nm ($E_{1\%}^1$=340), 367 nm ($E_{1\%}^1$=750), 386 nm ($E_{1\%}^1$=1280), and 410 nm ($E_{1\%}^1$=1430).

C. N-D,L-Lysyl amphotericin B methyl ester

A solution of N-D,L-lysyl amphotericin B (319 mg) in 5 ml of dimethylsulfoxide under nitrogen is treated with an excess of ethereal diazomethane. When a complete reaction is indicated by thin layer chromatography ($SiO_2$ eluted with the lower phase of a 1:1:1 chloroform:methanol:concentrated ammonium hydroxide mixture), the excess diazomethane is removed by a nitrogen stream and the resulting solution is diluted with 5 ml of methanol. Dropwise addition to excess ether yields a yellow precipitate which is filtered, washed with ether and dried to yield N-D,L-lysyl amphotericin B methyl ester; $\nu$ max (KBr) 1720 $cm^{-1}$; $[\alpha]_D^{26}$+290.9 (DMSO); $\lambda$ max (20% aqueous tetrahydrofuran) 348 nm ($E_{1\%}^1$=340), 367 nm ($E_{1\%}^1$=770), 386 nm ($E_{1\%}^1$=1300), 410 nm ($E_{1\%}^1$=1450).

Example 4

N-L-Lysyl amphotericin B methyl ester

A. N,N'-Bis-trichloroethoxycarbony-L-lysyloxy succinimide

To L-lysine (2.92 g) in 60 ml of methanol is added trichloroethyl-N-hydroxysuccinimidyl carbonate (14.55 g). The mixture is stirred overnight at room temperature, then treated with 2.5 ml concentrated ammonium hydroxide and stirred another 0.5 hour. The solvent is evaporated *in vacuo*

and the residue is extracted with water and ethyl acetate. The organic layer is extracted successively with cold 5% HCl, water and brine solution, and then is dried over $MgSO_4$. Filtration and evaporation produces N,N'-bis-trichloroethoxycarbonyl-L-lysine as a glass. An analytical sample is prepared by two precipitations from chloroform/hexane; $[\alpha]_D^{26}$—7.6° (EtOH); $\nu$ max (KBr) 1720 $cm^{-1}$.

In a manner similar to Example 2A, utilizing anhydrous ethyl acetate as the solvent, N,N'-bis-trichloroethoxycarbonyl-L-lysine is reacted with N-hydroxy succinimide to obtain the above named compound; $[\alpha]_D^{26}$—19.6° (EtOH); $\nu$ max (KBr) 1820, 1785, 1715 $cm^{-1}$.

B. N-(N',N''-Bis-trichloroethoxycarbonyl-L-lysyl) amphotericin B

To a stirred solution of amphotericin B (923 mg) in 10 ml dimethylsulfoxide and 1 ml of methanol under nitrogen is added 8.6 ml of a 0.21 *M* solution of the product of process step A in tetrahydrofuran. Sufficient dimethylsulfoxide is added to redissolve the precipitate formed and the solution is stirred for 5 hours. Dilution with an equal volume of methanol and dropwise addition in 300 ml ether gives a yellow precipitate. Filtration, washing with ether and drying gives the named compound: $[\alpha]_D^{26}$+198.0° (DMSO); $\lambda$ max (KBr) 1720 $cm^{-1}$.

C. N-(L-Lysyl)amphotericin B

To N-(N',N''-bis-trichloroethoxycarbony-L-lysyl) amphotericin B (1.04 g) in 40 ml of a 1:1 methanol:dimethylformamide mixture and (0.5 ml) acetic acid is added activated zinc dust (2.2 g). After 6.5 hours another 2.2 g zinc dust is added. After 8 hours the mixture is decanted into 500 ml ether. Filtration, washing with ether and drying gives a yellow powder. Chromatography on 135 g $SiO_2$ eluted with the lower phase of a 1:1:1 chloroform:methanol:concentrated ammonium hydroxide mixture produces the named compound: $\nu$ max (KBr) broad, 3600—2250 $cm^{-1}$; 1690 $cm^{-1}$ broad; $\lambda$ max (20% aqueous tetrahydrofuran), 348 nm ($E_{1\%}^1$=230), 367 nm ($E_{1\%}^1$=510), 386 nm ($E_{1\%}^1$=860) and 410 nm ($E_{1\%}^1$=930); $[\alpha]_D^{26}$+225.9° (DMSO).

D. N-L-Lysyl amphotericin B methyl ester

To N-L-lysyl amphotericin B (185 mg) in 10 ml of a 9:1 dimethylsulfoxide:methanol mixture is added excess ethereal diazomethane. When a complete reaction is indicated by thin layer chromatography ($SiO_2$, 1:1:1 chloroform:methanol:concentrated ammonium hydroxide), the mixture is treated with one drop of a 1:1 acetic acid:methanol solution and diluted with 10 ml of methanol. The solution is added dropwise to excess ether which is filtered, collected, washed with ether and dried to yield the named compound: $\lambda$ max (20% aqueous tetrahydrofuran) 348 nm ($E_{1\%}^1$=297), 367 nm ($E_{1\%}^1$=646), 386 nm ($E_{1\%}^1$=1095), 410 nm ($E_{1\%}^1$=1222).

Example 5

N-D-Lysyl amphotericin B methyl ester dihydrochloride

A. N,N'-Bis-(9-fluorenylmethyloxycarbonyl)-D-lysine

To a stirred solution of D-lysine hydrochloride (4.5 g) in 175 ml of a sodium carbonate solution (10% in water):acetone (6:1) solution is added dropwise a solution of 9-fluorenylmethyl azidoformate in acetone (150 ml). After 16 hours the mixture is diluted with water and washed three times with hexane. The mixture is then acidified with concentrated hydrochloric acid and extracted three times with ethyl acetate. The ethyl acetate solution is washed successively with dilute hydrochloric acid, water, saturated sodium bicarbonate solution, and brine, dried over magnesium sulfate, then concentrated *in vacuo* to about 20 ml. The solution is added to hexane (1 li) and the precipitate is collected and dried to afford the title compound: NMR (dimethylsulfoxide) $\delta$ 1.0—1.9 (m, 6H), 2.7—3.2 (m, 2H), 3.8—4.7 (m, 7H), 6.8—8.3 (m, 19H).

B. N,N'-Bis-(9-fluorenylmethyloxycarbonyl)-D-lysyloxy succinimide

To a stirred solution of N,N'-bis-(9-fluorenylmethyloxycarbonyl)-D-lysine (12.0 g) in 360 ml of an ethylacetate: dimethylformamide (35:1) mixture are added N-hydroxysuccinimide (3.1 g), 1-ethyl-3-(3-dimethylaminopropyl)carbodiimide hydrochloride (5.2 g) and diisopropylethylamine (2.0 ml). After 15 hours the solution is washed successively with dilute hydrochloric acid, water, saturated sodium bicarbonate solution, and brine, dried over magnesium sulfate, and concentrated *in vacuo* to about 20 ml. The solution is added to hexane (1 li) and the precipitate is collected and dried to afford the title compound.

C. N-[N',N''-Bis-(9-fluorenylmethyloxycarbonyl)-D-lysyl] amphotericin B

To a solution of N,N'-bis-(9-fluorenylmethyloxycarbonyl)-D-lysyloxy succinimide (0.60 g) in 22 ml of a dimethylformamide:dimethylsulfoxide:methanol (8:2:1) mixture is added amphotericin B (0.50 g). The solution is stirred at 40° for 2.5 hours, diluted with methanol (10 ml), and added to ether (500 ml). The yellow precipitate is filtered, washed with ether, and dried, Chromatography on $SiO_2$ (30 g) eluted with the lower phase of a 2:2:1 chloroform:methanol:water mixture produces the named compound.

D. N-[N',N''-Bis-(9-fluorenylmethyloxycarbonyl)-D-lysyl] amphotericin B methyl ester

To a stirred solution of N-[N',N''-bis-(9-fluorenylmethyloxycarbonyl)-D-lysyl]amphotericin B (1.4 g) in 55 ml of a dimethylsulfoxide:methanol (10:1) mixture are added methyliodide (1.6 ml) and diisopropylethylamine (1.2 ml). After 1 hour the solution is diluted with methanol (25 ml) and added to ether (1.5 l). The yellow precipitate is filtered and washed successively with ether, water, methanol:water (1:1) mixture, ether, and then dried to provide the title compound.

E. N-D-Lysyl amphotericin B methyl ester dihydrochloride

To a stirred solution of N-[N',N''-bis-(9-fluorenylmethyloxycarbonyl)-D-lysyl] amphotericin B methyl ester (1.2 g) in 50 ml of a dimethylsulfoxide:methanol (10:1) mixture is added 1,5-diazabicyclo[5.4.0]undec-5-ene (0.35 ml). After 0.5 hour the solution is diluted with methanol (25 ml) and added to ether (1 li). The yellow precipitate is filtered, washed with ether, and dried. The yellow powder is suspended in water (100 ml) and 1.0 n-hydrochloric acid is added until a pH of 5.5 is reached. The solution is filtered through a 0.22 $\mu$ micropore filter. The filtrate is lyophilized to afford the title compound: $\lambda$ max (20% aqueous tetrahydrofuran (348 nm ($E^1_{1\%}$=278), 367 nm ($E^1_{1\%}$=608), 387 nm ($E^1_{1\%}$=1023), 410 nm ($E^1_{1\%}$=1146).

Example 6

N-Glycyl derivative of the polyene macrolide antibiotic 67-121-C

A. N-Iodoacetyl derivative of the polyene macrolide antibiotic 67-121-C

67-121-C (600 mg) is dissolved in 10 ml of a 9:1 dimethylsulfoxide:methanol mixture. This solution is chilled and to it iodoacetic anhydride (180 mg) is added. The reaction is stirred at room temperature. Completion of the reaction is checked by thin layer chromatography (chloroform-methanol-water 2:2:1—lower phase). The resulting solution is diluted with an equal volume of methanol and added to vigorously stirred ether. The precipitate is filtered to yield the named compound.

B. N-Glycyl derivative of the polyene macrolide antibiotic 67-121-C

The product of step A is dissolved in a solution of dimethylformamide (10 ml) and concentrated ammonium hydroxide (28%; 3 ml). The reaction mixture is stirred for 2 hours and the volume is reduced to remove the aqueous ammonia. The residue is diluted with an equal volume of methanol and added to vigorously stirred ether. The precipitate is filtered and chromatographed on a silica gel column (chloroform:methanol:water (2:2:1)—lower phase) to obtain the title compound: $[\alpha]_D^{26}$+117° (dimethylsulfoxide); $\lambda$ max (20% aqueous tetrahydrofuran) 382 nm ($E^1_{1\%}$=765), 405 ($E^1_{1\%}$=660).

Example 7

N-($\beta$-Aminoethylglycyl)amphotericin B methyl ester

To 30 ml of a 9:1 dimethylsulfoxide:methanol mixture and 2.46 ml of ethylenediamine is added N-iodoacetyl amphotericin B, which may be prepared in a manner similar to Example 6A, over 2.25 hours. The mixture is stirred 1.5 hours and then diluted with an equal volume of methanol. The solution is added to vigorously stirred 500 ml of ether. The precipitate is filtered and washed with ether to yield N-($\beta$-aminoethylglycyl)amphotericin B. In a manner similar to that described in former Examples this compound can be transformed to the corresponding methylester: $\nu$ max (KBr) 1715 $cm^{-1}$; $\lambda$ max (20% aqueous tetrahydrofuran (348 nm ($E^1_{1\%}$=296), 367 nm ($E^1_{1\%}$=639), 386 nm ($E^1_{1\%}$=1074), 410 nm ($E^1_{1\%}$=1167).

In a manner analogous to those described above also other compounds of this invention can be prepared e.g. by treating amphotericin B, ascocin, aureofacin, aureofungin A, aureofungin B, candicidin, candidin, eurotin A, flavomycin, fulvomicin A, fulvomicin B, fulvomicin C, fungimycin, hamycin A, lucensomycin, levorin, mycoheptin, nystatin A, partricin, pimaricin, polyfungin, rimocidin, trichomycin A, vacidin A, DJ-400 $B_1$, DJ-400 $B_2$, 67-121A, 67-121B, 67-121C, PA 150, PA 616, 2814 H with the appropriate derivative of the desired amino compound such as glycine, lysine, glycylglycine, ornithine and ethanolamine or by transforming said polyene macrolide antibiotics into their respective N-iodoacetyl derivatives and reacting these derivatives with the appropriate amino compound such as ammonia, ethylenediamine and methylamine to obtain the respective N-glycyl, N-$\beta$-aminoethylglycyl or N-methylglycyl derivative of the relevant polyene macrolide antibiotic. Esters and acid addition salts of this invention are prepared according to known methods, especially according to the Examples.

The following compounds have been prepared accordingly:

N-D-lysyl-pimaricin methyl ester dihydrochloride, N-(2-aminoethoxycarbonyl)amphotericin B methyl ester, $[\alpha]_D^{26}$+281.9 (dimethylsulfoxide); $\lambda$ max (20% aqueous tetrahydrofuran) 348 nm ($E^1_{1\%}$=327), 367 nm ($E^1_{1\%}$=715), 386 nm ($E^1_{1\%}$=1206), 410 nm ($E^1_{1\%}$=1341),

N-$\beta$-alanyl amphotericin B methyl ester hydrochloride,

N-L-histidyl amphotericin B methyl ester dihydrochloride, $\lambda$ max (20% aqueous tetrahydrofuran): 348 nm ($E^1_{1\%}$=257), 367 nm ($E^1_{1\%}$=556), 387 nm ($E^1_{1\%}$=935), 410 nm ($E^1_{1\%}$=1036),

N-D-tryptophyl amphotericin B methyl ester hydrochloride, $\lambda$ max (20% aqueous tetrahydrofuran): 348 nm ($E^1_{1\%}$=315), 367 nm ($E^1_{1\%}$=693), 387 nm ($E^1_{1\%}$=1145), 410 nm ($E^1_{1\%}$=1230),

N-D,L-2,3-diaminopropionyl amphotericin B methyl ester dihydrochloride, $\lambda$ max (20% aqueous tetrahydrofuran): 348 nm ($E^1_{1\%}$=304), 367 nm ($E^1_{1\%}$=668), 387 nm ($E^1_{1\%}$=1128), 410 nm ($E^1_{1\%}$=1264),

N-D,L-2,4-diaminobutyryl amphotericin B methyl ester dihydrochloride, $\lambda$ max (20% aqueous tetrahydrofuran): 348 nm ($E^1_{1\%}$=307), 367 nm ($E^1_{1\%}$=676), 387 nm ($E^1_{1\%}$=1148), 410 nm ($E^1_{1\%}$=1292),

N-D-2,4-diaminobutyryl amphotericin B methyl ester,

N-L-2,4-diaminobutyryl amphotericin B methyl ester dihydrochloride, $\lambda$ max (20% aqueous tetrahydrofuran): 348 nm ($E^1_{1\%}$=319), 367 nm ($E^1_{1\%}$=702), 387 nm ($E^1_{1\%}$=1188), 410 nm ($E^1_{1\%}$=1340),

N-D,L-2,4-diaminobutyryl amphotericin B propargyl ester dihydrochloride, $\lambda$ max (20% aqueous tetrahydrofuran): 348 nm ($E^1_{1\%}$=294), 367 nm ($E^1_{1\%}$=635), 387 nm ($E^1_{1\%}$=1069), 410 nm ($E^1_{1\%}$=1192),

N-D,L-ornithyl amphotericin B hydrochloride, $\lambda$ max (20% aqueous tetrahydrofuran) 366 nm ($E^1_{1\%}$=740), 386 nm ($E^1_{1\%}$=1260), 400 nm ($E^1_{1\%}$=1400),

N-D,L-ornithyl amphotericin B methyl ester, $\lambda$ max (20% aqueous tetrahydrofuran) 365 nm ($E^1_{1\%}$=590), 386 nm ($E^1_{1\%}$=990), 401 nm ($E^1_{1\%}$=1100),

N-L-ornithyl amphotericin B methyl ester dihydrochloride, $\lambda$ max (20% aqueous tetrahydrofuran): 348 nm ($E^1_{1\%}$=300), 367 nm ($E^1_{1\%}$=667), 387 nm ($E^1_{1\%}$=1124), 410 nm ($E^1_{1\%}$=1251),

N-D-ornithyl amphotericin B methyl ester dihydrochloride, $\lambda$ max (20% aqueous tetrahydrofuran): 348 nm ($E^1_{1\%}$=315), 367 nm ($E^1_{1\%}$=692), 387 nm ($E^1_{1\%}$=1171), 410 nm ($E^1_{1\%}$=1312),

N-D-ornithyl amphotericin B propargyl ester dihydrochloride, $\lambda$ max (20% aqueous tetrahydrofuran): 348 nm ($E^1_{1\%}$=270), 367 nm ($E^1_{1\%}$=574), 387 nm ($E^1_{1\%}$=957), 410 nm ($E^1_{1\%}$=1054),

N-D-ornithyl amphotericin B (3-phenyl-2-propynyl) ester dihydrochloride, $\lambda$ max (20% aqueous tetrahydrofuran): 348 nm ($E^1_{1\%}$=254), 367 nm ($E^1_{1\%}$=540), 387 nm ($E^1_{1\%}$=905), 410 nm ($E^1_{1\%}$=986),

N-D-$\beta$-lysyl amphotericin B methyl ester dihydrochloride, $\lambda$ max (20% aqueous tetrahydrofuran): 348 nm ($E^1_{1\%}$=291), 367 nm ($E^1_{1\%}$=636), 387 nm ($E^1_{1\%}$=1075), 410 nm ($E^1_{1\%}$=1205),

N-L-$\beta$-lysyl amphotericin B methyl ester dihydrochloride, $\lambda$ max (20% aqueous tetrahydrofuran): 348 nm ($E^1_{1\%}$=290), 367 nm ($E^1_{1\%}$=640), 387 nm ($E^1_{1\%}$=1080), 410 nm ($E^1_{1\%}$=1205),

N-glycylglycyl amphotericin B, $\lambda$ max (20% aqueous tetrahydrofuran) 367 nm ($E^1_{1\%}$=760), 386 nm ($E^1_{1\%}$=1280), 401 nm ($E^1_{1\%}$=1430),

N-glycylglycyl amphotericin B methyl ester, $\lambda$ max (20% aqueous tetrahydrofuran) 367 nm ($E^1_{1\%}$=710), 386 nm ($E^1_{1\%}$=1200), 401 nm ($E^1_{1\%}$=1340),

N-(N',N''-diglycyl-D-lysyl) amphotericin B methyl ester dihydrochloride, $\lambda$ max (20% aqueous tetrahydrofuran): 348 nm ($E^1_{1\%}$=263), 367 nm ($E^1_{1\%}$=578), 387 nm ($E^1_{1\%}$=1012), 410 nm ($E^1_{1\%}$=1094),

N-D-lysyllysyl amphotericin B methyl ester,

N-D-2,4-diaminobutyryl amphotericin B methyl ester dihydrochloride $[\alpha]^{26}_D$+256.6° (dimethylsulfoxide),

N-D-2,4-diaminobutyryl amphotericin B propargyl ester dihydrochloride $[\alpha]^{26}_D$+228.4° (dimethylsulfoxide).

The following formulations exemplify the formulations in which the compounds of this invention can be used. The formulations are prepared by methods known in the art.

## Formulations

### Formulation 1

### Cream

| | mg/g |
|---|---|
| N-glycyl amphotericin B methyl ester hydrochloride | 50.0 |
| white petrolatum | 150.0 |
| mineral oil | 60.0 |
| cetyl alcohol | 36.0 |
| stearyl alcohol | 36.0 |
| cetomacrogal 1000 | 22.0 |
| 4-chlor-m-cresol | 1.0 |
| purified water to make | 1 g. |

### Formulation 2

### Ointment

| | mg/g |
|---|---|
| N-glycyl amphotericin B methyl ester hydrochloride | 10.0 |
| propylene glycol | 100 |
| propylene glycol monostearate | 20.0 |
| white wax | 60.0 |
| white petrolatum, sufficient to make | 1 g. |

Formulation 3
Lotion

| | mg/ml |
|---|---|
| N-glycyl amphotericin B methyl ester hydrochloride | 50.0 |
| isopropyl myristate | 20.0 |
| glycerol stearate | 2.0 |
| promalgen 6 | 3.0 |
| propylene glycol | 15.0 |
| purified water, sufficient to make | 1 ml |

Formulation 4
Aqueous suspension for intramuscular or subcutaneous injection

| | mg/ml |
|---|---|
| N-glycyl amphotericin B methyl ester | 70.0—100.1 |
| polysorbate 80, USP | 0.5 |
| benzyl alcohol | 9.0 |
| sodium chloride | 5.0 |
| methylparaben | 1.3 |
| propylparaben | 0.2 |
| sodium carboxymethylcellulose | 5.0 |
| disodium edetate | 0.1 |
| polyethylene glycol | 20.0 |
| water for injection, sufficient to make | 1.0 ml. |

Formulation 5
Intravenous injection
(Supplied as sterile lyophilized powder)

| | mg/vial |
|---|---|
| N-glycyl amphotericin B methyl ester hydrochloride | 40.0—60.0 |
| sodium desoxycholate | 70.0 |
| dibasic sodium phosphate (anhydrous) | 16.0 |
| monobasic sodium phosphate (anhydrous) | 9.0 |

Formulation 6
Oral suspension
(to give a dose of 25 mg./5 ml.)

| | |
|---|---|
| N-glycyl amphotericin B methyl ester hydrochloride | 5.00 g. |
| Magnesium aluminum silicate | 9.5 g. |
| Sodium carboxymethylcellulose, U.S.P. | 2.5 g. |
| Sodium citrate, U.S.P. | 25.0 g. |
| Flavor | q.s. |
| Color | q.s. |
| Methylparaben, U.S.P. | 0.9 g. |
| Propylparaben, U.S.P. | 0.2 g. |
| Polysorbate 80, U.S.P. | 1.0 g. |
| Sorbitol Solution, U.S.P. | 500.0 g. |
| Water q.s. | 1000.0 g. |

Procedure:

1. Heat 200 ml. of water to boiling, and dissolve in it one half of the parabens. Cool to about 70°C, then mix in the Polysorbate 80. Sprinkle in the silicate, stirring until a uniform smooth suspension results.

2. Heat an additional 200 ml. of water to boiling, and dissolve in it the remainder of the parabens. Disperse the carboxymethylcellulose in this until a smooth gel results. Mix in the Sorbitol Solution. Then introduce and dissolve the sodium citrate.

3. Add 2 to 1 slowly, with constant stirring. Cool the mixture to 25°C. Add the N-glycyl amphotericin G methyl ester hydrochloride, flavor and color, mixing thoroughly. Add sufficient quantity of water to make the total volume 1000 ml.

**Claims for the Contracting States BE, CH, DE, FR, GB, IT, NL, SE.**

1. Polyene macrolide antibiotic derivatives of the general formula

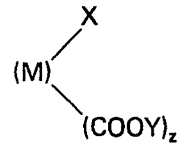

(I)

wherein M is a polyene macrolide antibiotic moiety containing an amino sugar moiety, the amino group of which is substituted by X, wherein X is

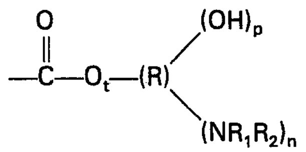

(II),

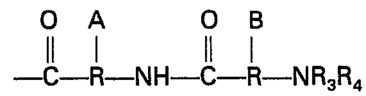

(III),

or

$—R_5$

wherein A and B independently of each other are hydrogen or $NR_3R_4$ or one of A and B is hydrogen and the other is

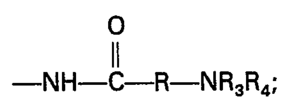

R is a saturated hydrocarbon group having 1 to 10 carbon atoms; $R_1$ and $R_2$ independently of each other are hydrogen or a straight chain alkyl group having 1 to 3 carbon atoms or one of $R_1$ and $R_2$ is hydrogen and the other a straight chain alkyl group having 1 to 3 carbon atoms substituted by an $NH_2$ group; $R_3$ and $R_4$ independently of each other are hydrogen or a straight chain alkyl group containing 1 to 3 carbon atoms; $R_5$ is histidyl, tyrosyl, tryptophyl, arginyl, phenylalanyl or prolyl or an

$(\alpha)—N—[(NH_2)_m—(R)—CO]$-derivative

of histidyl, tyrosyl, tryptophyl, phenylalanyl or prolyl; m is 1 or 2; n is 1 to 3, t is zero or 1, and when t is zero p is zero to 4 and when t is 1 p is zero; with the proviso that not more that one hetero atom is attached to the same carbon atom in any one of R, $R_1$ and $R_2$ and that in those compounds containing more than one R-group, these R-groups may be of the same or different chain length;

z is zero or 1, and

Y is hydrogen, an alkyl group containing 1 to 10 carbon atoms, allyl, propargyl, benzyl or phenylpropargyl, and the pharmaceutically acceptable acid addition salts of all the foregoing compounds.

2. A compound according to claim 1 which is derived from a polyene macrolide antibiotic of the general formula

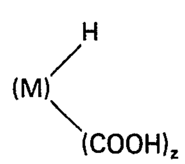

(IV),

wherein z is zero or 1, which polyene macrolide antibiotic is amphotericin B, ascocin, aureofacin, aureofungin A, aureofungin B, candicidin, candidin, eurotin A, flavomycin, fulvomicin A, fulvomicin B, fulvomicin C, fungimycin, hamycin A, lucensomycin, levorin, mycoheptin, nystatin A, partricin,

pimaricin, polyfungin, rimocidin, trichomycin A, vacidin A, DJ-400 $B_1$, DJ-400 $B_2$, 67-121A, 67-121B, 67-121C, PA 150, PA 616, or 2814 H, preferably from amphotericin B, pimaricin or 67-121C.

3. A compound according to claim 1 or 2, wherein X is

$$-\overset{\overset{O}{\|}}{C}-(R)-(NR_1R_2)_n \qquad (V),$$

wherein n is 1 or 2 and R, $R_1$ and $R_2$ are as defined in claim 1.

4. A compound according to any one of claims 1 to 3 wherein R is a saturated hydrocarbon group having 1 to 5 carbon atoms and $R_1$, $R_2$, $R_3$ and $R_4$ are hydrogen.

5. A compound according to any one of claims 1 to 4 wherein z is 1 and Y is methyl.

6. A compound according to claim 1 which is N-D-lysyl amphotericin B methyl ester, N-D-ornithyl amphotericin B methyl ester, N-D-lysyl pimaricin methyl ester, N-glycyl 67-121C, N-glycyl amphotericin B methyl ester, N-D-2,4-diaminobutyryl amphotericin B methyl ester, N-$\beta$-alanyl amphotericin B methyl ester, N-(aminoethylglycyl) amphotericin B, N-(aminoethylglycyl) amphotericin B methyl ester, N-D-lysyl amphotericin B, N-L-lysyl amphotericin B, N-L-lysyl amphotericin B methyl ester, N-L-ornithyl amphotericin B methyl ester, N-D-ornithyl amphotericin B propargyl ester, N-(2-aminoethoxycarbonyl) amphotericin B methyl ester, N-L-histidyl amphotericin B methyl ester, or a pharmaceutically acceptable acid addition salt, especially the hydrochloride salt, thereof.

7. Process for the preparation of a polyene macrolide antibiotic derivative of the general formula

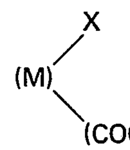

(I)

wherein M, X and z are as defined in claim 1, and Y is hydrogen, an alkyl group containing 1 to 10 carbon atoms, allyl, propargyl, benzyl or phenylpropargyl, or a pharmaceutically acceptable acid addition salt thereof, characterized in that either one of the following processes is applied:

a) for the preparation of a compound of the general formula I, defined in claim 1: reaction of a polyene macrolide antibiotic of the general formula

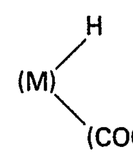

(VI),

wherein M, Y and z are as defined in claim 1, is reacted with a reactive derivative of a compound of the general formula HOX' (VII), wherein X' is defined as group x in claim 1 with the exception that any free amino group contained therein is protected, followed by the removal of the protecting group(s) and/or esterification of compounds containing a carboxyl group;

b) for the preparation of a derivative of a polyene macrolide antibiotic of the general formula

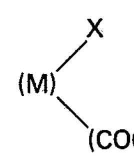

(I),

wherein M is a polyene macrolide antibiotic moiety containing an amino sugar moiety, the amino group of which is substituted by X, wherein X is

$$-CO-CH_2-NHR_1 \qquad (X),$$

wherein $R_1$ is hydrogen, or a straight chain alkyl group having 1 to 3 carbon atoms which may be substituted by an $NH_2$ group; z is zero or 1, and

Y is hydrogen, an alkyl group containing 1 to 10 carbon atoms, allyl, propargyl, benzyl or phenylpropargyl: reaction of a N-iodoacetyl derivative of the polyene macrolide antibiotic with an amine of the general formula $H_2NR_1$ (XI), wherein $R_1$ is as defined above and, followed if desired, by esterification where the macrolide contains a carboxyl group,

and if desired the so obtained product is transformed into a pharmaceutically acceptable acid addition salt.

8. A compound of the general formula (I) as defined in any one of claims 1 to 6 or obtained by a process of claim 7 for use in treating fungal infections.

9. A pharmaceutical composition comprising as active ingredient at least one compound of the general formula (I) as defined in any one of claims 1 to 6 or obtained by a process of claim 7 together with a pharmaceutically acceptable carrier.

10. A composition according to claim 9 for use in treating fungal infections.

**Claims for the Contracting State AT**

1. Process for the preparation of polyene macrolide antibiotic derivatives of the general formula

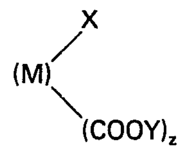

(I)

wherein M is a polyene macrolide antibiotic moiety containing an amino sugar moiety, the amino group of which is substituted by X, wherein X is

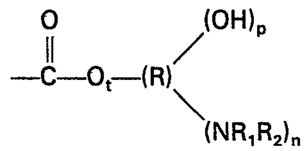

(II),

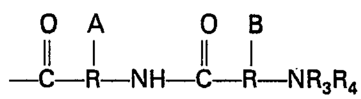

(III)

or

$$—R_5$$

wherein A and B independently of each other are hydrogen or $NR_3R_4$ or one of A and B is hydrogen and the other is

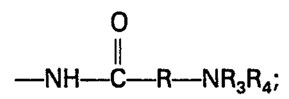

R is a saturated hydrocarbon group having 1 to 10 carbon atoms; $R_1$ and $R_2$ independently of each other are hydrogen or a straight chain alkyl group having 1 to 3 carbon atoms or one of $R_1$ and $R_2$ is hydrogen and the other a straight chain alkyl group having 1 to 3 carbon atoms substituted by an $NH_2$ group; $R_3$ and $R_4$ independently of each other are hydrogen or a straight chain alkyl group containing 1 to 3 carbon atoms; $R_5$ is histidyl, tyrosyl, tryptophyl, arginyl, phenylalanyl or propyl or an

$$(\alpha)—N—[(NH_2)_m—(R)—CO]\text{-derivative}$$

of histidyl, tyrosyl, tryptophyl, phenylalanyl or prolyl; m is 1 or 2; n is 1 to 3, t is zero or 1, and when t is zero p is zero to 4 and when t is 1 p is zero; with the proviso that not more than one hetero atom is attached to the same carbon atom in any one of R, $R_1$ and $R_2$ and that in those compounds containing more than one R-group, these R-groups may be of the same or different chain length;

z is zero or 1, and

Y is hydrogen, an alkyl group containing 1 to 10 carbon atoms, allyl, propargyl, benzyl or phenylpropargyl; and the pharmaceutically acceptable acid addition salts of all the foregoing compounds, characterized in that either one of the following processes is applied:

a) for the preparation of a compound of the general formula (I), wherein M is a polyene macrolide antibiotic moiety containing an amino sugar moiety, the amino group of which is substituted by X, wherein X is

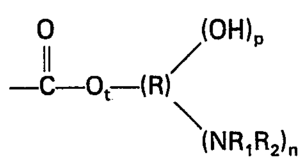

(II),

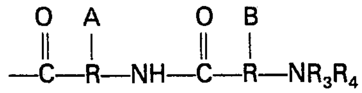

(III),

or

—$R_5$

wherein A and B independently of each other are hydrogen or $NR_3R_4$ or one of A and B is hydrogen and the other is

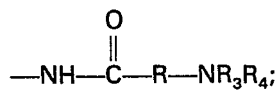

R is a saturated hydrocarbon group having 1 to 10 carbon atoms; $R_1$ and $R_2$ independently of each other are hydrogen or a straight chain alkyl group having 1 to 3 carbon atoms or one of $R_1$ and $R_2$ is hydrogen and the other a straight chain alkyl group having 1 to 3 carbon atoms substituted by an $NH_2$ group; $R_3$ and $R_4$ independently of each other are hydrogen or a straight chain alkyl group containing 1 to 3 carbon atoms; $R_5$ is histidyl, tyrosyl, tryptophyl, arginyl, phenylalanyl or prolyl or an

$(\alpha)$—N—[$(NH_2)_m$—(—R—)—CO]-derivative

of histidyl, tyrosyl, tryptophyl, phenylalanyl or prolyl; m is 1 or 2; n is 1 to 3, t is zero or 1, and when t is zero p is zero to 4 and when t is 1 p is zero; with the proviso that not more than one hetero atom is attached to the same carbon atom in any one of R, $R_1$ and $R_2$ and that in those compounds containing more than one R-group, these R-groups may be of the same or different chain length;

z is zero or 1, and

Y is hydrogen, an alkyl group containing 1 to 10 carbon atoms, allyl, propargyl, benzyl or phenylpropargyl: reaction of a polyene macrolide antibiotic of the general formula

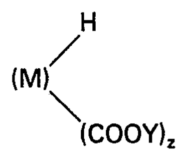

(VI),

wherein M, Y and z are as defined above, is reacted with a reactive derivative of a compound of the general formula HOX′ (VII), wherein X′ is defined as group X above with the exception that any free amino group contained therein is protected, followed by the removal of the protecting group(s) and/or esterification of compounds containing a carboxyl group;

b) for the preparation of a compound of the general formula (I), wherein M is a polyene macrolide antibiotic moiety containing an amino sugar moiety, the amino group of which is substituted by X, wherein X is

$$—CO—CH_2—NHR_1 \quad (X),$$

wherein $R_1$ is hydrogen, or a straight chain alkyl group having 1 to 3 carbon atoms which may be substituted by an $NH_2$ group; z is zero or 1, and Y is hydrogen, an alkyl group containing 1 to 10 carbon atoms, allyl, propargyl, benzyl or phenylpropargyl: reaction of a N-iodoacetyl derivative of the polyene macrolide antibiotic with an amine of the general formula $H_2NR_1$ (XI), wherein $R_1$ is as defined above and, followed if desired, by esterification provided the macrolide contains a carboxyl group; and if desired the so obtained product is transformed into a pharmaceutically acceptable acid addition salt.

2. Process according to claim 1 characterized in that the polyene antibiotic starting material is selected from amphotericin B, ascocin, aureofacin, aureofungin A, aureofungin B, candicidin, candidin, eurotin A, flavomycin, fulvomicin A, fulvomicin B, fulvomicin C, fungimycin, hamycin A, lucensomycin, levorin, mycoheptin, nystatin A, partricin, pimaricin, polyfungin, rimocidin, trichomycin A, vacidin A, DJ-400 $B_1$, DJ-400 $B_2$, 67-121A, 67-121B, 67-121C, PA 150, PA 616 and 2814 H, preferably from amphotericin B, pimaricin and 67-121C.

3. Process according to claim 1a or 2 characterized in that for the preparation of compounds of formula I, wherein z is one and Y is an alkyl group containing 1 to 10 carbon atoms, allyl, propargyl, benzyl or phenylpropargyl, a polyene macrolide antibiotic of formula VI, wherein M is as defined in claim 1, Y is hydrogen and z is one, is reacted with a compound ZX′, wherein X′ is as defined in claim 1, the amino group(s) being protected by fluorenemethoxycarbonyl group(s), and Z is N-hydroxysuccinimidyl, which reaction is followed by esterification and then by removal of the protecting group(s).

4. Process according to any one of claims 1 to 3, characterized in that a compound of formula I is prepared wherein X is

$$-\overset{\overset{\displaystyle O}{\|}}{C}\!-\!(\!-R\!-\!)\!-\!(NR_1R_2)_n \qquad (V),$$

wherein n is 1 or 2 and R, $R_1$ and $R_2$ are as defined in claim 1, R preferably being a saturated hydrocarbon group having 1 to 5 carbon atoms.

5. Process according to any one of claims 1 to 4 characterized in that a compound of formula I is prepared wherein $R_1$, $R_2$, $R_3$ and $R_4$ are hydrogen.

6. Process according to any one of claims 1 to 5, characterized in that a compound of formula I is prepared wherein X is lysyl, ornithyl, histidyl or tryptophyl.

7. Process according to any one of claims 1 to 6, characterized in that a compound of formula I is prepared wherein z is 1 and Y is an alkyl group containing 1 to 4 carbon atoms, preferably being methyl.

8. Process according to claim 1, characterized in that a compound of formula I is prepared which is N-D-lysyl amphotericin B methyl ester, N-D-ornithyl amphotericin B methyl ester, N-D-lysyl pimaricin methyl ester, N-glycyl 67-121C, N-glycyl amphotericin B methyl ester, N-D-2,4-diaminobutyryl amphotericin B methyl ester, N-$\beta$-alanyl amphotericin B methyl ester, N-(aminoethylglycyl) amphotericin B, N-(aminoethylglycyl) amphotericin B methyl ester, N-D-lysyl amphotericin B, N-L-lysyl amphotericin B, N-L-lysyl amphotericin B methyl ester, N-L-ornithyl amphotericin B methyl ester, N-D-ornithyl amphotericin B propargyl ester, N-(2-aminoethoxycarbonyl) amphotericin B methyl ester, N-L-histidyl amphotericin B methyl ester, or a pharmaceutically acceptable acid addition salt, especially the hydrochloride salt, thereof.

9. Process for the preparation of a pharmaceutical composition comprising as active ingredient at least one compound of the general formula (I) as defined in claim 1, characterized in that the said active ingredient is brought into a form suitable for therapeutic administration.

10. Process as claimed in claim 9, characterized in that a compound of the formula I prepared by a process as claimed in any one of claims 1 to 8 is admixed with a pharmaceutical carrier or excipient.

**Patentansprüche für die Vertragsstaaten: BE, CH, DE, FR, GB, IT, NL, SE**

1. Polyen-Makrolid-Antibiotikum-Derivate der allgemeinen Formel

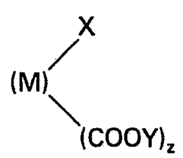

(I),

in der M eine Polyen-Makrolid-Antibiotikum-Struktureinheit ist, die eine Aminozucker-Struktureinheit enthält, deren Amino-Gruppe durch X substituiert ist, wobei X

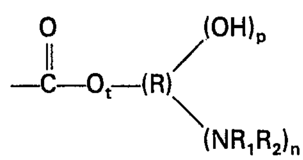

(II),

$$-\overset{\overset{\displaystyle O}{\|}}{C}-\overset{\overset{\displaystyle A}{|}}{R}-NH-\overset{\overset{\displaystyle O}{\|}}{C}-\overset{\overset{\displaystyle B}{|}}{R}-NR_3R_4 \qquad (III)$$

oder

$$-R_5, \qquad \text{ist,}$$

worin

A und B unabhängig voneinander Wasserstoff oder $NR_3R_4$ sind oder einer der Substituenten A und B Wasserstoff ist und der andere

$$-NH-\overset{\overset{\displaystyle O}{\|}}{C}-R-NR_3R_4 \text{ ist,}$$

R eine gesättigte Kohlenwasserstoff-Gruppe mit 1 bis 10 Kohlenstoff-Atomen ist,

$R_1$ und $R_2$ unabhängig voneinander Wasserstoff oder eine geradkettige Alkyl-Gruppe mit 1 bis 3 Kohlenstoff-Atomen sind oder einer der Substituenten $R_1$ und $R_2$ Wasserstoff ist under der andere eine durch eine $NH_2$-Gruppe substituierte geradkettige Alkyl-Gruppe mit 1 bis 3 Kohlenstoff-Atomen ist,

$R_3$ und $R_4$ unabhängig voneinander Wasserstoff oder eine geradkettige Alkyl-Gruppe mit 1 bis 3 Kohlenstoff-Atomen sind,

$R_5$ Histidyl, Tyrosyl, Tryptophyl, Arginyl, Phenylalanyl oder Prolyl oder ein

$$(\alpha)\text{—}N\text{—}[(NH_2)_m\text{-}(R)\text{-}CO]\text{-Derivat}$$

von Histidyl, Tyrosyl, Tryptophyl, Phenylalanyl oder Prolyl ist,

m 1 oder 2 ist,

n 1 bis 3 ist,

t 0 oder 1 ist und

p 0 bis 4 ist, wenn t 0 ist, und p 0 ist, wenn t 1 ist,

mit der Maßgabe, daß nicht mehr als ein Hetero-Atom an das gleiche Kohlensoff in irgendeinem der Reste R, $R_1$ und $R_2$ gebunden ist und daß in den Verbindungen, die mehr als eine R-Gruppe enthalten, diese R-Gruppen die gleich oder eine unterschiedliche Kettenlänge besitzen können,

z 0 oder 1 ist und

Y Wasserstoff, eine Alkyl-Gruppe mit 1 bis 10 Kohlenstoff-Atomen, Allyl, Propargyl, Benzyl oder Phenylpropargyl ist,

sowie die pharmazeutisch unbedenklichen Säureadditions salze sämtlicher der vorgenannten Verbindungen.

2. Verbindung nach Anspruch 1, abgeleitet von einem Polyen-Makrolid-Antibiotikum der allgemeinen Formel

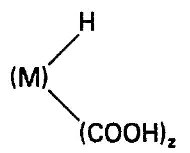

(IV),

in der

z 0 oder 1 ist, wobei das Polyen-Makrolid-Antibiotikum Amphotericin B, Ascocin, Aureofacin, Aureofungin A, Aureofungin B, Candicidin, Candidin, Eurotin A, Flavomycin, Fulvomicin A, Fulvomicin B, Fulvomicin C, Fungimycin, Hamycin A, Lucensomycin, Levorin, Mycoheptin, Nystatin A, Partricin, Pimaricin, Polyfungin, Rimocidin, Trichomycin A, Vacidin A, DJ-400 $B_1$, DJ-400 $B_2$, 67-121A, 67-121B, 67-121C, PA 150, PA 616 oder 2814 H, vorzugsweise Amphotericin B, Pimaricin Oder 67-121C, ist.

3. Verbindung nach Anspruch 1 oder 2, dadurch gekennzeichnet, daß X

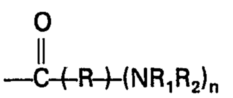

(V)

ist, worin n 1 oder 2 ist und R, $R_1$ und $R_2$ die in Anspruch 1 angegebenen Bedeutungen haben.

4. Verbindung nach Anspruch 1 bis 3, dadurch gekennzeichnet, daß R eine gesättigte Kohlenwasserstoff-Gruppe mit 1 bis 5 Kohlenstoff-Atomen ist und $R_1$, $R_2$, $R_3$ und $R_4$ Wasserstoff sind.

5. Verbindung nach Anspruch 1 bis 4, dadurch gekennzeichnet, daß z 1 ist und Y Methyl ist.

6. Verbindung nach Anspruch 1

N-D-Lysyl-amphotericin B-methylester,
N-D-Ornithyl-amphotericin B-methylester,
N-D-Lysyl-pimaricin-methylester,
N-Glycyl-67-121C,
N-Glycyl-amphotericin B-methylester,
N-D-2,4-Diaminobutyryl-amphotericin B-methylester,
N-ß-Alanyl-amphotericin B-methylester,
N-(Aminoethylglycyl)-amphotericin B,
N-(Aminoethylglycyl)-amphotericin B-methylester,
N-D-Lysyl-amphotericin B,
N-L-Lysyl-amphotericin B,
N-L-Lysyl-amphotericin B-methylester,

N-L-Ornithyl-amphotericin B-methylester,
N-D-Ornithyl-amphotericin B-propargylester,
N-(2-Aminoethoxycarbonyl)-amphotericin B-methylester,
N-L-Histidyl-amphotericin B-methylester,

sowie ihre pharmazeutisch unbedenklichen Säureadditionssalze, insbesondere ihre Hydrochlorid-Salze.

7. Verfahren zur Herstellung eines Polyen-Makrolid-Antibiotikum-Derivats der allgemeinen Formel

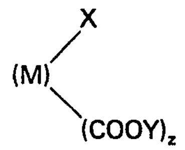

(I),

in der M, X und z die in Anspruch 1 angegebenen Bedeutungen haben und Y Wasserstoff, eine Alkyl-Gruppe mit 1 bis 10 Kohlenstoff-Atomen, Allyl, Propargyl, Benzyl oder Phenylpropargyl ist, oder eines pharmazeutisch unbedenklichen Säureadditionssalzes desselben, dadurch gekennzeichnet, daß eines der nachstehenden Verfahren angewandt wird:

(a) für die Herstellung einer Verbindung der allgemeinen Formel I nach Anspruch 1: Reaktion eines Polyen-Makrolid-Antibiotikums der allgemeinen Formel

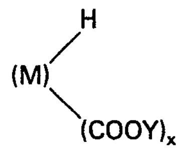

(VI),

in der

M, Y und z die in Anspruch 1 angegebenen Bedeutungen haben, mit einem reaktionsfähigen Derivat einer Verbindung der allgemeinen Formel

$$HOX' \qquad (VII),$$

in der X' definiert ist wie die Gruppe X in Anspruch 1, jedoch mit der Abweichung, daß jede darin enthaltene freie Amino-Gruppe geschützt ist, gefolgt von der Entfernung der Schutzgruppe (n) und/oder Veresterung von Verbindungen, die eine Carboxyl-Gruppe enthalten;

b) für die Herstellung eines Derivats eines Polyen-Makrolid-Antibiotikums der allgemeinen Formel

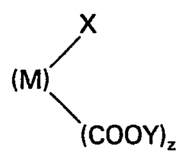

(I),

in der M eine Polyen-Makrolid-Antibiotikum-Sturktureinheit ist, die eine Aminozucker-Struktureinheit enthält, deren Amino-Gruppe durch X substituiert ist, wobei X

$$—CO—CH_2—NHR_1 \qquad (X)$$

ist, worin

$R_1$ Wasserstoff oder eine geradkettige Alkyl-Gruppe mit 1 bis 3 Kohlenstoff-Atomen ist, die durch eine $NH_2$-Gruppe substituiert sein kann,
z null oder 1 ist und
Y Wasserstoff, eine Alkyl-Gruppe mit 1 bis 10 Kohlenstoff-Atomen, Allyl, Propargyl, Benzyl oder Phenylpropargyl ist:

Reaktion eines N-Iodoacetyl-Derivats des Polyen-Makrolid-Antibiotikums mit einem Amin der allgemeinen Formel

$$H_2NR_1 \qquad (XI),$$

in der $R_1$ die im Vorstehenden angegebene Bedeutung hat, und, falls erwünscht, nachfolgende Veresterung, sofern das Makrolid eine Carboxy-Gruppe enthält, und, falls erwünscht, Überführung des so erhaltenen Produkts in ein pharmazeutisch unbedenkliches Säureadditionssalz.

8. Verbindung der allgemeinen Formel (I) nach Anspruch 1 bis 6 oder erhalten durch ein Verfahren nach Anspruch 7 zur Verwendung bei der Behandlung von Pilz-Infektionen.

9. Pharmazeutische Zusammensetzung, enthaltend als Wirkstoff mindestens eine Verbindung der allgemeinen Formel (I) nach Anspruch 1 bis 6 oder erhalten durch ein Verfahren nach Anspruch 7 zusammen mit einem pharmazeutisch unbedenklichen Träger.

10. Zusammensetzung nach Anspruch 9 zur Verwendung bei der Behandlung von Pilz-Infektionen.

**Patentansprüche für der Vertragsstaat: AT**

1. Verfahren zur Herstellung von Polyen-Makrolid-Antibiotikum-Derivaten der allgemeinen Formel

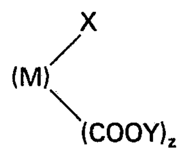

(I),

in der M eine Polyen-Makrolid-Antibiotikum-Struktureinheit ist, die eine Aminozucker-Struktureinheit enthält, deren Amino-Gruppe durch X substituiert ist, wobei X

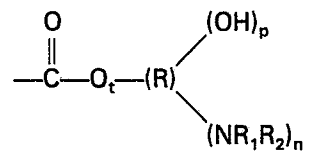

(II),

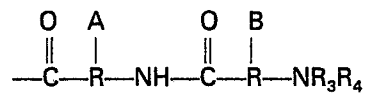

(III)

oder

$$-R_5,$$

ist, worin

A und B unabhängig voneinander Wasserstoff oder $NR_3R_4$ sind oder einer der Substituenten A und B Wasserstoff ist und der andere

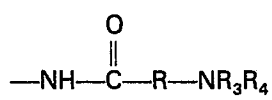

ist,

R eine gesättigte Kohlenwasserstoff-Gruppe mit 1 bis 10 Kohlenstoff-Atomen ist,
$R_1$ und $R_2$ unabhängig voneinander Wasserstoff oder eine geradkettige Alkyl-Gruppe mit 1 bis 3 Kohlenstoff-Atomen sind oder einer der Substituenten $R_1$ und $R_2$ Wasserstoff ist und der andere eine durch eine $NH_2$-Gruppe substituierte geradkettige Alkyl-Gruppe mit 1 bis 3 Kohlenstoff-Atomen ist,
$R_3$ und $R_4$ unabhängig voneinander Wasserstoff oder eine geradkettige Alkyl-Gruppe mit 1 bis 3 Kohlenstoff-Atomen sind,
$R_5$ Histidyl, Tyrosyl, Tryptophyl, Arginyl, Phenylalanyl oder Prolyl oder ein

$$(\alpha)-N-[(NH_2)_m-(R)-CO]\text{-Derivat}$$

von Histidyl, Tyrosyl, Tryptophyl, Phenylalanyl oder Prolyl ist,
m 1 oder 2 ist,
n 1 bis 3 ist,
t 0 oder 1 ist und
p 0 bis 4 ist, wenn t 0 ist, und p 0 ist, wenn t 1 ist,

mit der Maßgabe, daß nicht mehr als ein Hetero-Atom an das gleiche Kohlenstoff in irgendeinem der

Reste R, $R_1$ und $R_2$ gebunden ist und daß in den Verbindungen, die mehr als eine R-Gruppe enthalten, diese R-Gruppen die gleiche oder eine unterschiedliche Kettenlänge besitzen können,
z 0 oder 1 ist und
Y Wasserstoff, eine Alkyl-Gruppe mit 1 bis 10 Kohlenstoff-Atomen, Allyl, Propargyl, Benzyl oder Phenylpropargyl ist,

sowie der pharmazeutisch unbedenklichen Säureadditionssalze sämtlicher der vorgenannten Verbindungen, dadurch gekennzeichnet, daß eines der nachstehenden Verfahren angewandt wird:

a) für die Herstellung einer Verbindung der allgemeinen Formel (I), in der M eine Polyen-Makrolid-Antibiotikum-Struktureinheit ist, die eine Aminozucker-Struktureinheit enthält, deren Amino-Gruppe durch X substituiert ist, wobei X

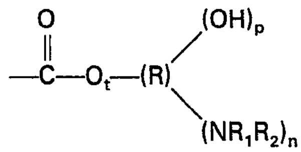
(II),

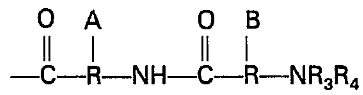
(III)

oder

$$-R_5,$$

ist, worin
A und B unabhängig voneinander Wasserstoff oder $NR_3R_4$ sind oder einer der Substituenten A und B Wasserstoff ist und der andere

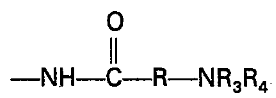

ist,

R eine gesättigte Kohlenwasserstoff-Gruppe mit 1 bis 10 Kohlenstoff-Atomen ist,1
$R_1$ und $R_2$ unabhängig voneinander Wasserstoff oder eine geradkettige Alkyl-Gruppe mit 1 bis 3 Kohlenstoff-Atomen sind oder einer der Substituenten $R_1$ und $R_2$ Wasserstoff ist und der andere eine durch eine $NH_2$-Gruppe substituierte geradkettige Alkyl-Gruppe mit 1 bis 3 Kohlenstoff-Atomen ist,
$R_3$ und $R_4$ unabhängig voneinander Wasserstoff oder eine geradkettige Alkyl-Gruppe mit 1 bis 3 Kohlenstoff-Atomen sind,
$R_5$ Histidyl, Tyrosyl, Tryptophyl, Arginyl, Phenylalanyl oder Prolyl oder ein

$$(\alpha)-N-[(NH_2)_m-(R)-CO]\text{-Derivat}$$

von Histidyl, Tyrosyl, Tryptophyl, Phenylalanyl oder Prolyl ist,
m 1 oder 2 ist,
n 1 bis 3 ist,
t 0 oder 1 ist und
p 0 bis 4 ist, wenn t 0 ist, und p 0 ist, wenn t 1 ist,

mit der Maßgabe, daß nicht mehr als ein Hetero-Atom an das gleiche Kohlenstoff in irgendeinem der Reste R, $R_1$ und $R_2$ gebunden ist und daß in den Verbindungen, die mehr als eine R-Gruppe enthalten, diese R-Gruppen die gleiche oder eine unterschiedliche Kettenlänge besitzen können,

z 0 oder 1 ist und
Y Wasserstoff, eine Alkyl-Gruppe mit 1 bis 10 Kohlenstoff-Atomen, Allyl, Propargyl, Benzyl oder Phenylpropargyl ist:

Reaktion eines Polyen-Makrolid-Antibiotikums der allgemeinen Formel

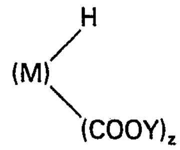

(VI),

in der

M, Y und z die im Vorstehenden angegebenen Bedeutungen haben, mit einem reaktionsfähigen Derivat einer Verbindung der allgemeinen Formel

$$HOX' \qquad (VII),$$

in der X' definiert ist wie die Gruppe X im Vorstehenden, jedoch mit der Abweichung, daß jede darin enthaltene freie Amino-Gruppe geschützt ist, gefolgt von der Entfernung der Schutzgruppe (n) und/oder Veresterung von Verbindungen, die eine Carboxyl-Gruppe enthalten;

b) für die Herstellung einer Verbindung der allgemeinen Formel (I), in der M eine Polyen-Makrolid-Antibiotikum-Struktureinheit ist, die eine Aminozucker-Strucktureinheit enthält, deren Amino-Gruppe durch X substituiert ist, wobei X

$$-CO-CH_2-NHR_1 \qquad (X)$$

ist, worin

$R_1$ Wasserstoff oder eine geradkettige Alkyl-Gruppe mit 1 bis 3 Kohlenstoff-Atomen ist, die durch eine $NH_2$-Gruppe substituiert sein kann,

z null oder 1 ist und

Y Wasserstoff, eine Alkyl-Gruppe mit 1 bis 10 Kohlenstoff-Atomen, Allyl, Propargyl, Benzyl oder Phenylpropargyl ist:

Reaktion eines N-Iodoacetyl-Derivats des Polyen-Makrolid-Antibiotikums mit einem Amin der allgemeinen Formel

$$H_2NR_1 \qquad (XI),$$

in der $R_1$ die im Vorstehenden angegebene Bedeutung hat, und, falls erwünscht nachfolgende Veresterung, sofern das Makrolid eine Carboxy-Gruppe enthält, und, falls erwünscht, Überführung des so erhaltenen Produkts in ein pharmazeutisch unbedenkliches Säureadditionssalz.

2. Verfahren nach Anspruch 1, dadurch gekennzeichnet, daß das Polyen-Antibiotikum-Ausgangsmaterial ausgewählt ist aus Amphotericin B, Ascocin, Aureofacin, Aureofungin A, Aureofungin B, Candicidin, Candidin, Eurotin A, Flavomycin, Fulvomicin A, Fulvomicin B, Fulvomicin C, Fungimycin, Hamycin A, Lucensomycin, Levorin, Mycoheptin, Nystatin A, Partricin, Pimaricin, Polyfungin, Rimocidin, Trichomycin A, Vacidin A, DJ-400 $B_1$, DJ-400 $B_2$, 67-121A, 67-121B, 67-121C, PA 150, PA 616 oder 2814 H, vorzugsweise aus Amphotericin B, Pimaricin oder 67-121C.

3. Verfahren nach Anspruch 1 oder 2, dadurch gekennzeichnet, daß zur Herstellung von Verbindungen der Formel I, in der z 1 ist und Y eine Alkyl-Gruppe mit 1 bis 10 Kohlenstoff-Atomen, Allyl, Propargyl, Benzyl oder Phenylpropargyl ist, ein Polyen-Makrolid-Antibiotikum der Formel (VI), in der M die in Anspruch 1 angegebene Bedeutung hat, Y Wasserstoff ist und z eins ist, mit einer Verbindung ZX', in der X' die in Anspruch 1 angegebene Bedeutung hat, wobei die Amino-Gruppe (n) durch die Fluorenmethoxycarbonyl-Gruppe (n) geschützt ist (sind), und Z N-Hydroxymethylsuccinimidyyl ist, umgesetzt wird, wobei im Anschluß an diese Reaktion die Veresterung und danach die Entfernung der Schutzgruppe (n) erfolgen.

4. Verfahren nach Anspruch 1 bis 3, dadurch gekennzeichnet, daß eine Verbindung der Formel I hergestellt wird, in der X

$$-\overset{O}{\overset{\|}{C}}-(R)-(NR_1R_2)_n \qquad (V)$$

ist, worin n 1 oder 2 ist und R, $R_1$ und $R_2$ die in Anspruch 1 angegebenen Bedeutungen haben, wobei R vorzugsweise eine gesättigte Kohlenwasserstoff-Gruppe mit 1 bis 5 Kohlenstoff-Atomen ist.

5. Verfahren nach Anspruch 1 bis 4, dadurch gekennzeichnet, daß eine Verbindung der Formel I hergestellt wird, in der $R_1$, $R_2$, $R_3$ und $R_4$ Wasserstoff sind.

6. Verfahren nach Anspruch 1 bis 5, dadurch gekennzeichnet, daß eine Verbindung der Formel I hergestellt wird, in der X Lysyl, Ornithyl, Histidyl oder Tryptophyl ist.

7. Verfahren nach Anspruch 1 bis 6, dadurch gekennzeichnet, daß eine Verbindung der Formel I hergestellt wird, in der z 1 ist und Y eine Alkyl-Gruppe mit 1 bis 4 Kohlenstoff-Atomen, vorzugsweise Methyl, ist.

8. Verfahren nach Anspruch 1 bis 7, dadurch gekennzeichnet, daß eine Verbindung der Formel I hergestellt wird, die

N-D-Lysyl-amphotericin B-methylester,
N-D-Ornithyl-amphotericin B-methylester,
N-D-Lysyl-pimaricin-methylester,
N-Glycyl-67-121C,
N-Glycyl-amphotericin B-methylester,
N-D-2,4-Diaminobutyryl-amphotericin B-methylester,
N-$\beta$-Alanyl-amphotericin B-methylester,
N-(Aminoethylglycyl)-amphotericin B,
N-(Aminoethylglycyl)-amphotericin B-methylester,
N-D-Lysyl-amphotericin B,
N-L-Lysyl-amphotericin B,
N-L-Lysyl-amphotericin B-methylester,
N-L-Ornithyl-amphotericin B-methylester,
N-D-Ornithyl-amphotericin B-propargylester,
N-(2-Aminoethoxycarbonyl)-amphotericin B-methylester,
N-L-Histidyl-amphotericin B-methylester,

oder eines ihrer pharmazeutisch unbedenklichen Säureadditionssalze, insbesondere ihrer Hydrochlorid-Salze, ist.

9. Verfahren zur Herstellung einer pharmazeutischen Zusammensetzung, die als Wirkstoff mindestens eine Verbindung der allgemeinen Forml (I) nach Anspruch 1 enhält, dadurch gekennzeichnet, daß der Wirkstoff in eine Form gebracht wird, die für eine therapeutische Verabreichung geeignet ist.

10. Verfahren nach Anspruch 9, dadurch gekennzeichnet, daß einer Verbindung der Formel I, die hergestellt wurde nach einem Verfahren der Ansprüche 1 bis 8, mit einem pharmazeutischen Träger oder Hilfsstoff vermischt wird.

**Revendications pour les Etats contractants: BE, CH, DE, FR, GB, IT, NL, SE**

1. Dérivés de polyène macrolide antibiotique de formule générale

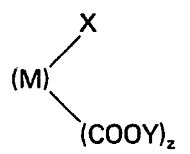

(I)

où M est un fragment de polyène macrolide antibiotique contenant un fragment de sucre aminé, dont le groupe amino est substitué par X, où X est

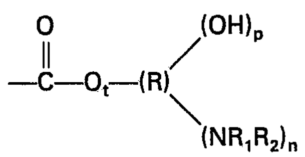

(II),

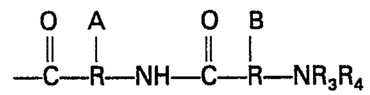

(III),

ou,

$$-R_5$$

où A et B sont, indépendamment l'un de l'autre, de l'hydrogène ou $NR_3R_4$ ou bien l'un de A et de B est de l'hydrogène et l'autre est

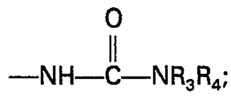

R est un groupe hydrocarbure saturé ayant de 1 à 10 atomes de carbone; $R_1$ et $R_2$ sont, indépendamment l'un de l'autre, de l'hydrogène ou un groupe alcoyle à chaîne droite ayant de 1 à 3 atomes de carbone ou bien l'un de $R_1$ et $R_2$ est de l'hydrogène et l'autre est un groupe alcoyle à chaîne droite ayant de 1 à 3 atomes de carbone substitué par un groupe $NH_2$; $R_3$ et $R_4$ sont, indépendamment l'un de l'autre, de l'hydrogène ou un groupe alcoyle à chaîne droite contenant de 1 à 3 atomes de carbone; $R_5$ est histidyle, tyrosyle, tryptophyle, arginyle, phénylalanyle ou prolyle ou bien un dérivé

$$(\alpha)—N—[(NH_2)_m—(R)—CO]$$

de l'histidyle du tyrosyle, du tryptophyle, du phénylalanyle ou du prolyle; *m* est égal 1 ou 2; *n* est 1 à 3, *t* est zéro ou 1 et quand *t* est zéro *p* est zéro à 4 et quand *t* est 1 *p* est zéro; à condition qu'il n'y ait pas plus d'un hétéro atome attaché au même atome de carbone dans chacun de R, $R_1$ et $R_2$ et que dans les composés contenant plus d'un groupe R, ces groupes R puissent avoir une longueur de chaîne identique ou différente;

*z* est zéro ou 1, et

Y est de l'hydrogène, un groupe alcoyle contenant de 1 à 10 atomes de carbone, allyle, propargyle, benzyle ou phénylpropargyle, et les sels d'addition d'acide acceptables en pharmacie de tous les composés ci-dessus.

2. Composé selon la revendication 1 qui est dérivé d'un polyène macrolide antibiotique de formule générale

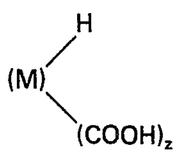
(IV),

où *z* est zéro ou 1, lequel polyène macrolide antibiotique est l'amphotéricine B, l'ascocine, l'auréofacine, l'auréofongine A, l'auréofongine B, la candicidine, la candidine, l'eurotine A, la flavomycine, la fulvomicine A, la fulvomicine B, la fulvomicine C, la fongimycine, l'hamycine A, la lucensomycine, la lévoríne, la mycoheptine, la nystatine A, la partricine, la pimaricine, la polyforgine, la rimocidine, la trichomycine A, la vacidine A, DJ-400 $B_1$, DJ-400 $B_2$, 67-121A, 67-121B, 67-121C, PA 150, PA 616 ou 2814 H, de préférence à partir d'amphotéricine B, de pimaricine ou de 67-121C.

3. Composé selon la revendication 1 ou 2 où X est

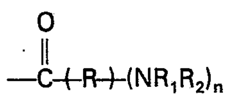
(V),

où *n* est 1 ou 2 et R, $R_1$ et $R_2$ sont tels que définis à la revendication 1.

4. Composé selon l'une quelconque des revendications 1 à 3, où R est un groupe hydrocarbure saturé ayant de 1 à 5 atomes de carbone et $R_1$, $R_2$, $R_3$ et $R_4$ sont de l'hydrogène.

5. Composé selon l'une quelconque des revendications 1 à 4, où *z* est 1 et Y est du méthyle.

6. Composé selon la revendication 1 qui est du méthyl ester de N-D-lysyl amphotéricine B, du méthyl ester de N-D-ornithyl amphotéricine B, du méthyl ester de N-D-lysyl pimaricine, du N-glycyl 67-121C, du méthyl ester de N-glycyl amphotéricine B, du méthyl ester de N-D-2,4-diaminobutyryl amphotéricine B, du méthyl ester de N-$\beta$-alanyl amphotéricine B, de la N-(aminoéthylglycyl)-amphotéricine B, du méthyl ester de N-(aminoéthylglycyl)amphotéricine B, de la N-D-lysyl amphotéricine B, de la N-L-lysyl amphotéricine B, du méthyl ester de N-L-lysyl amphotéricine B, du méthyl ester de N-L-ornithyl amphotéricine B, du propargyl ester de N-D-ornithyl amphotéricine B, du méthyl ester de N-(2-aminoéthoxycarbonyl)amphotéricine B, du méthyl ester de N-L-histidyl amphotéricine B, ou son sel d'addition d'acide acceptable en pharmacie, en particulier le sel de chlorhydrate.

7. Procédé de préparation d'un dérivé de polyène macrolide antibiotique de formule générale

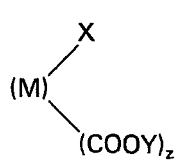
(I)

où M, X et *z* sont tels que définis à la revendication 1 et Y est de l'hydrogène, un groupe alcoyle

contenant de 1 à 10 atomes de carbone, de l'allyle, du propargyle, du benzyle ou du phénylpropargyle, ou bien un sel d'addition d'acide acceptable en pharmacie, caractérisé en ce que l'on applique l'un des procédés qui suivent:

a) pour la préparation d'un composé de formule générale I, défini selon la revendication 1: réaction d'un polyène macrolide antibiotique de formule générale

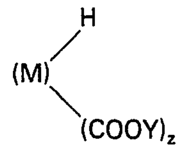 (VI),

où M, Y et *z* sont tels que définis à la revendication 1, avec un dérivé réactif d'un composé de formule générale HOX' (VII), où X' est défini par le groupe X de la revendication 1 à l'exception que tout groupe amino libre qui y est contenu est protégé, avec ensuite l'enlèvement du ou des groupes protecteurs et/ou l'estérification des composés contenant un groupe carboxyle;

b) pour la préparation d'un dérivé d'un polyène macrolide antibiotique de formule générale

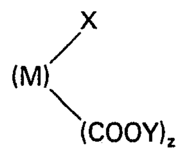 (I),

où M est un fragment de polyène macrolide antibiotique contenant un fragment de sucre aminé, dont le groupe amino est substitué par X, où X est

$$—CO—CH_2—NHR_1 \qquad (X),$$

où $R_1$ est de l'hydrogène ou un groupe alcoyle à chaîne droite ayant de 1 à 3 atomes de carbone qui peut être substitué par un groupe $NH_2$; *z* est zéro ou 1, et Y est de l'hydrogène, un groupe alcoyle contenant de 1 à 10 atomes de carbone, de l'allyle, du propargyle, du benzyle ou du phénylpropargyle:

réaction d'un dérivé de N-iodoacétyle du polyène macrolide antibiotique avec une amine de formule générale $H_2NR_1$ (XI) où $R_1$ est tel que défini ci-dessus et, avec ensuite si on le souhaite, l'estérification si le macrolide contient un groupe carboxyle,

et si on le souhaite, le produit ainsi obtenu est transformé en sel d'addition d'acide acceptable en pharmacie.

8. Composé de formule générale (I) selon l'une quelconque des revendications 1 à 6 ou obtenu par un procédé selon la revendication 7 pour une utilisation pour le traitement des infections dues aux champignons.

9. Composition pharmaceutique comprenant, comme ingrédient actif, au moins un composé de la formule générale (I) tel que défini selon l'une quelconque des revendications 1 à 6 ou obtenu par un procédé selon la revendication 7 avec un véhicule acceptable en pharmacie.

10. Composition selon la revendication 9, pour une utilisation pour le traitement d'infections dues aux champignons.

**Revendications pour l'Etat Contractant: AT**

1. Procédé de préparation de dérivés de polyène macrolide antibiotique de formule générale

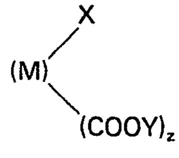 (I)

où M est un fragment de polyène macrolide antibiotique contenant un fragment de sucre aminé, dont le groupe amino est substitué par X, où X est

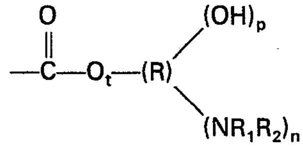 (II),

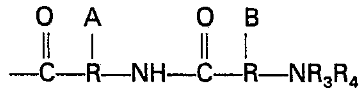

(III)

ou

—$R_5$

où A et B sont indépendamment l'un de l'autre de l'hydrogène ou $NR_3R_4$ ou bien l'un de A et B est de l'hydrogène et l'autre est

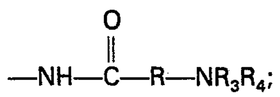

R est un groupe hydrocarbure saturé ayant de 1 à 10 atomes de carbone; $R_1$ et $R_2$ sont, indépentamment l'un de l'autre, de l'hydrogène ou un groupe alcoyle à chaîne droite ayant de 1 à 3 atomes de carbone ou bien l'un de $R_1$ et $R_2$ est de l'hydrogène et l'autre est un groupe alcoyle à chaîne droite ayant de 1 à 3 atomes de carbone substitué par un groupe $NH_2$; $R_3$ et $R_4$ sont, indépendamment l'un de l'autre, de l'hydrogène ou un groupe alcoyle à chaîne droite contenant de 1 à 3 atomes de carbone; $R_5$ est hystidyle, tyrosyle, tryptophyle, arginyle, phénylalanyle ou propyle ou un dérivé

$(\alpha)$—N—$[(NH_2)_m$—(R)—CO]

d'hystidyle, de tyrosyle, de tryptophyle, de phénylalanyle ou de prolyle; *m* est 1 ou 2; *n* est 1 à 3; *t* est zéro-ou 1 et quend *t* est zéro *p* est zéro à 4 et quand *t* est 1 *p* est zéro; à condition qu'il n'y ait pas plus d'un hétéroatome attaché au même atome de carbone dans chacun de R, $R_1$ et $R_2$ et que dans les composés contenant plus d'un groupe R, ces groupes R puissent avoir une longueur de chaîne identique ou différente;

*z* est zéro ou 1, et

Y est de l'hydrogène, un groupe alcoyle contenant de 1 à 10 atomes de carbone, de l'allyle, du propargyle, du benzyle ou du phénylpropargyle; et les sels d'addition d'acide acceptables en pharmacie de tous les composés ci-dessus, caractérisé en ce qu'on applique l'un des procédés qui suivent:

a) pour la préparation d'un composé de formule générale (I), où M est un fragment de polyène macrolide antibiotique contenant un fragment de sucre aminé, dont le groupe amino est substitué par X, où X est

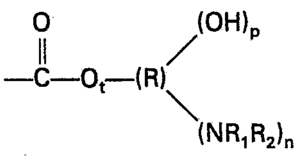

(II),

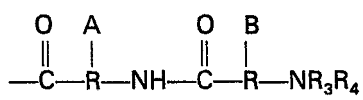

(III),

ou

—$R_5$,

où A et B sont indépendamment l'un de l'autre de l'hydrogène ou $NR_3R_4$ ou bien l'un de A et B est de l'hydrogène et l'autre est

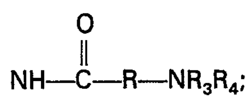

R est un groupe hydrocarbure saturé ayant de 1 à 10 atomes de carbone; $R_1$ et $R_2$ sont, indépendamment l'un de l'autre, de l'hydrogène ou un groupe alcoyle à chaîne droite ayant de 1 à 3 atomes de carbone ou bien l'un de $R_1$ et $R_2$ est de l'hydrogène et l'autre est un groupe alcoyle à chaîne droite ayant de 1 à 3 atomes de carbone substitué par un groupe $NH_2$; $R_3$ et $R_4$ sont, indépendamment l'un de l'autre, de l'hydrogène ou un groupe alcoyle à chaîne droite contenant de 1 à 3 atomes de carbone; $R_5$ est hystidyle, tyrosyle, tryptophyle, arginyle, phénylalanyle ou prolyle ou bien un dérivé

$(\alpha)$—N—$[(NH_2)_m$—(R)—CO]

d'histidyle, tyrosyle, tryptophyle, phénylalanyle ou prolyle; *m* est 1 ou 2; *n* est 1 à 3, *t* est zéro ou 1 et quand *t* est zéro *p* est zéro à 4 et quand *t* est 1 *p* zéro; à condition qu'il n'y ait pas plus d'un hétéroatome attaché au même atome de carbone dans chacun de R, $R_1$ et $R_2$ et que dans les composés contenant plus d'un groupe R, ces groupes R puissent avoir une longueur de chaîne identique ou différente;

*z* est zéro ou 1, et

Y est de l'hydrogène, un groupe alcoyle contenant de 1 à 10 atomes de carbone, de l'allyle, du propargyle, du benzyle ou du phénylpropargyle:

la réaction d'un polyène macrolide antibiotique de formule générale

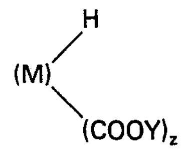

(VI),

où M, Y et *z* sont tels que définis ci-dessus qui réagit avec un dérivé réactif d'un composé de formule générale HOX' (VII), où X' est défini par le groupe X ci-dessus à l'exception que tout groupe amino libre qui y est contenu est protégé, avec ensuite enlèvement du ou des groupes protecteurs et/ou estérification des composés contenant un groupe carboxyle;

b) pour la préparation d'un composé de formule générale (I) où M est un fragment de polyène macrolide antibiotique contenant un fragment de sucre aminé, dont le groupe amino est substitué par X, où X est

$$-CO-CH_2-NHR_1 \qquad (X),$$

où $R_1$ est de l'hydrogène, ou un groupe alcoyle à chaîne droite ayant de 1 à 3 atomes de carbone qui peut être substitué par un groupe $NH_2$; *z* est zéro ou 1, et Y est de l'hydrogène, un groupe alcoyle contenant de 1 à 10 atomes de carbone, de l'allyle, du propargyle, du benzyle ou du phénylpropargyle: la réaction d'un dérivé de N-iodoacétyle du polyène macrolide antibiotique avec une amine de formule générale $H_2NR_1$ (XI), où $R_1$ est tel que défini ci-dessus et, ensuite, si on le souhaite, l'estérification à condition que le macrolide contienne un groupe carboxyle; et si on le souhaite, le produit ainsi obtenu est transformé en un sel d'addition d'acide acceptable en pharmacie.

2. Procédé selon la revendication 1, caractérisé en ce que le polyène antibiotique comme matière première est choisi parmi l'amphotéricine B, l'ascocine, l'auréofacine, l'auréofongine A, l'auréofongine B, la candicidine, la condidine, l'eurotine A, la flavomycine, la fulvomicine A, la fulvomicine B, la fulvomicine C, la fongimycine, l'hamycine A, la lucensomycine, la lévorine, la mycoheptine, la nystatine A, la partricine, la pimaricine, la polyfongine, la rimocidine la trychomycine A, la vacidine A, DJ-400 $B_1$, DJ-400 $B_2$, 67-121A, 67-121B, 67-121C, PA 150, PA 616 et 2814 H, de préférence parmi l'amphotéricine B, la pimaricine et 67-121C.

3. Procédé selon la revendication 1a ou 2, caractérisé en ce que pour la préparation des composés de formule I, où *z* est 1 et Y est un groupe alcoyle contenant de 1 à 10 atomes de carbone, de l'allyle, du propargyle, du benzyle ou du phénylpropargyle, on fait réagir un polyène macrolide antibiotique de formule VI, où *M* est tel qui défini à la revendication 1, Y est de l'hydrogène et *z* est 1, avec un composé ZX', où X' est tel que défini à la revendication 1, le ou les groupes amino étant protégés par un ou des groupes fluorèneméthoxycarbonyles, et Z est N-hydroxysuccinimidyle, laquelle réaction est suivie d'une estérification puis de l'enlèvement du ou des groupes protecteurs.

4. Procédé selon l'une quelconque des revendications 1 à 3, charactérisé en ce qu'un composé de formule I est préparé où X est

$$-\overset{\overset{\displaystyle O}{\|}}{C}-(-R-)-(NR_1R_2)_n \qquad (V),$$

où *n* est 1 ou 2 et R, $R_1$ et $R_2$ sont tels que définis à la revendication 1, R étant de préférence un groupe hydrocarbure saturé ayant de 1 à 5 atomes de carbone.

5. Procédé selon l'une quelconque des revendications 1 à 4, caractérisé en ce qu'un composé de formule I est préparé dans lequel $R_1$, $R_2$, $R_3$ et $R_4$ sont de l'hydrogène.

6. Procédé selon l'une quelconque des revendications 1 à 5, caractérisé en ce qu'un composé de formule I est préparé où X est lysyle, ornithyle, histidyle ou tryptophyle.

7. Procédé selon l'une quelconque des revendications précédentes, caractérisé en ce qu'un composé de formule I est préparé où *z* est 1 et Y est un groupe alcoyle contenant de 1 à 4 atomes de carbone, de préférence du méthyle.

8. Procédé selon la revendication 1, caractérisé en ce qu'un composé de formule I est préparé qui est du méthyl ester de N-D-lysyl amphotéricine B, du méthyl ester de N-D-ornithyl amphotéricine B, du

méthyl ester de N-D-lysyl pimaricine, du N-glycyl 67-121C, du méthyl ester de N-glycyl amphotéricine B, du méthyl ester de N-D-2,4-diaminobutyryl amphotéricine B, du méthyl ester de N-$\beta$-alanyl amphotéricine B, de la N-(aminoéthylglycyl)amphotéricine B, du méthyl ester de N-(aminoéthylglycyl)-amphotéricine B, de le N-D-lysyl amphotéricine B, de la N-L-lysyl amphotéricine B, du méthyl ester de N-L-lysyl amphotéricine B, du méthyl ester de N-L-ornithyl amphotéricine B, du propargyl ester de N-D-ornithyl amphotéricine B, du méthyl ester de (N-(2-aminoéthoxycarbonyl)amphotéricine B, du méthyl ester de N-L-hystidyl amphotéricine B ou un sel d'addition d'acide acceptable en pharmacie, en particulier le sel de chlorhydrate.

9. Procédé de préparation d'une composition pharmaceutique comprenant comme ingrédient actif, au moins un composé de formule générale (I) selon la revendication 1, caractérisé en ce que ledit ingrédient actif est amené sous une forme appropriée à une administration thérapeutique.

10. Procédé selon la revendication 9, caractérisé en ce qu'un composé de formule I préparé par le procédé selon l'une quelconque des revendications 1 à 8 est mélange à un véhicule ou excipient pharmaceutique.